# EUROPÄISCHE PATENTSCHRIFT

(11) **EP 1 086 077 B1**
(45) Veröffentlichungstag und Bekanntmachung des Hinweises auf die Patenterteilung: **07.04.2004**
(21) Anmeldenummer: 99931059.2
(22) Anmeldetag: 09.06.1999
(51) Int. Cl.: C07C 317/18, C07C 323/12, C07D 303/34, C07C 43/23, C07C 215/08, C07D 295/08, C07D 303/18, A61K 31/10, A61K 31/335, A61K 31/085, A61K 31/135, A61K 31/45

(54) **NEUE ANTIESTROGENE, VERFAHREN ZU IHRER HERSTELLUNG UND IHRE PHARMAZEUTISCHE VERWENDUNG**
NOVEL ANTIESTROGENS, A METHOD FOR THE PRODUCTION THEREOF, AND THEIR PHARMACEUTICAL APPLICATION
NOUVEAUX ANTI-ESTROGENES, LEUR PROCEDE DE PRODUCTION ET LEUR UTILISATION PHARMACEUTIQUE

(30) Priorität: 09.06.1998 DE 19826213
(43) Veröffentlichungstag der Anmeldung: 28.03.2001
(73) Patentinhaber: Schering Aktiengesellschaft, 13353 Berlin (DE)
(72) Erfinder: KLAR, Ulrich, D-13503 Berlin (DE); BOHLMANN, Rolf, D-14055 Berlin (DE); PARCZYK, Karsten, D-12207 Berlin (DE); FRITZEMEIER, Karl-Heinrich, D-13503 Berlin (DE); LESSL, Monika, D-13467 Berlin (DE); LICHTNER, Rosemarie, D-10823 Berlin (DE)
(86) Internationale Anmeldenummer: PCT/EP1999/004049
(87) Internationale Veröffentlichungsnummer: WO 1999/064393

(56) Entgegenhaltungen:
- FR-A- 2 685 332
- J. LU, ET AL.: "Antioestrogenic activity of two 11beta-oestradiol derivatives on MCF-7 breast cancer cells" STEROIDS: STRUCTURE, FUNCTION, AND REGULATION, Bd. 60, Nr. 8, 1. August 1995 (1995-08-01), Seiten 512-518, XP004026503 Elsevier Science Publishers, New York, NY, US ISSN: 0039-128X

## Beschreibung

Die vorliegende Erfindung betrifft 3,4-Diphenyl-bicyclo[4.3.0]nonyl-Derivate der allgemeinen Formel l 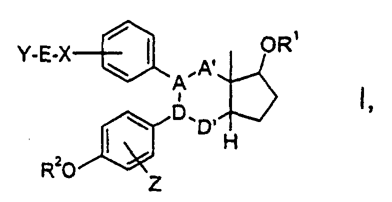 worin
- R¹: für gegebenenfalls substituiertes C₁-C₂₀-Alkanoyl, gegebenenfalls substituiertes C₁-C₂₀-Alkyl, gegebenenfalls substituiertes C₇-C₂₀-Aralkyl, gegebenenfalls substituiertes C₇-C₁₅-Aroyl, eine Gruppe PG¹ oder ein Wasserstoffatom,
- R²: für gegebenenfalls substituiertes C₁-C₂₀-Alkanoyl, gegebenenfalls substituiertes C₁-C₂₀-Alkyl, gegebenenfalls substituiertes C₇-C₂₀-Aralkyl, gegebenenfalls substituiertes C₇-C₁₅-Aroyl, eine Gruppe PG² oder ein Wasserstoffatom,
- PG¹, PG²: gleich oder verschieden sind und für eine Schutzgruppe PG,
- A'-A-D-D': für eine -CH₂-C(OH)-C=CH-, -CH=C-C(OH)-CH₂-, -CH=C-C=CH-, -CH₂-C=C-CH₂-, -CH₂-CH-CH-CH₂-, -CH₂-C(OH)-CH-CH₂-, -CH₂-CH-C(OH)-CH₂-, -CH₂-C(OH)-C(OH)-CH₂- oder 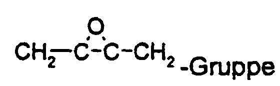 (Hydroxy= α oder β; Epoxy = α oder β),
- X: für eine Bindung, ein Sauerstoffatom, ein Schwefelatom, SO oder SO₂,
- E: für eine gerad- oder verzweigtkettige Alkylen-, Alkenylen- oder Alkinylengruppe mit bis zu 15 Kohlenstoffatomen,
- Y: für Halogen (F, Cl, Br, I), für einen Substituenten R⁴, einen gegebenenfalls substituierten Aryl- oder Heteroarylrest, eine NR^{4a}R^{4b}, SO₂NR^{4a}R^{4b}-, NR^{4a}(CH₂)ₚ-Q-G-, NR⁵(CHR⁶-CHR⁷)-(CH₂)ₜ-Q-G-, SO₂NR^{4a}(CH₂)ₚ-Q-G-, eine O-G-, S-G-. SO-G-, SO₂-G-Gruppe,
- R⁴: für ein Wasserstoffatom, gegebenenfalls substituiertes C₁-C₂₀-Alkyl, teilweise oder vollständig fluoriertes C₁-C₂₀-Alkyl, gegebenenfalls substituiertes C₁-C₂₀-Alkanoyl, gegebenenfalls substituiertes Aryl, gegebenenfalls substituiertes Heteroaryl, gegebenenfalls substituiertes C₇-C₂₀-Aralkyl, gegebenenfalls substituiertes C₇-C₁₅-Aroyl,
- Q: für ein Sauerstoffatom, ein Schwefelatom, SO oder SO₂
- G: für -(CH₂)ₙ-R³,
- n: für 0 bis 10,
- p: für 1 bis 10,
- t: für 0,1 oder 2
- R³: für Wasserstoff, eine gerad- oder verzweigtkettige Alkyl-, Alkenyl- oder Alkinylgruppe mit bis zu 10 Kohlenstoffatomen, eine gerad- oder verzweigtkettige, teilweise oder vollständig fluorierte Alkyl- oder Alkenylgruppe mit bis zu 10 Kohlenstoffatomen, eine gegebenenfalls substituierte C₄-C₈-Cycloalkyl-, eine gegebenenfalls substituierte Aryl-, eine gegebenenfalls substituierte C₇-C₂₀-Aralkylgruppe oder, falls n>0 ist, auch für eine Hydroxygruppe oder ein Halogenatom,
- R^{4a}, R^{4b}: gleich oder verschieden sind in der Bedeutung von R⁴ oder gemeinsam für eine C₃-C₁₅-Alkylengruppe, die geradkettig oder verzweigt sein kann,
- R⁵: ein Wasserstoffatom oder eine C₁₋₅-Alkylgruppe,
- R⁶ und R⁷: je ein Wasserstoffatom, oder
- R⁵ und R⁶: gemeinsam eine Alkylengruppe ―(CH₂)_{d}― mit d = 2, 3, 4 oder 5 und R⁷ ein Wasserstoffatom oder
- R⁵ und R⁷: gemeinsam eine Alkylengruppe ―(CH₂)ₑ―mit e = 2, 3 oder 4 und R⁶ ein Wasserstoffatom,
- Z: für Wasserstoff, Halogen, OH, N₃, NH₂, CO₂H, CO₂-(C₁-C₂₀)-Alkyl, C₁-C₂₀-Alkoxy, -NO₂, -CN, oder C₁-C₂₀-Acyloxy,
stehen.

Die Erfindung betrifft die Diastereomeren und/oder Enantiomeren dieser Derivate und auch deren Gemische.

Als Alkylgruppen R¹, R², R³ und R⁴ sind gerad- oder verzweigtkettige Alkylgruppen mit bis zu 20 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R¹, R², R³ und R⁴ können teilweise oder vollständig fluoriert oder substituiert sein durch 1-10 Halogenatome, Hydroxygruppen, C₁-C₄-Alkoxygruppen, C₆-C₁₂-Arylgruppen, die durch 1-3 Halogenatome substituiert sein können, Di-(C₁-C₄)-Alkylamine und Tri-(C₁-C₄)Alkylammonium.
Im Falle einer gerad- oder verzweigtkettigen, teilweise oder vollständig fluorierten Alkylgruppe handelt es sich vorzugsweise um die Trifluormethyl- oder oder Pentafluorethylgruppe.
Als Cycloalkylgruppen R³ kommen substituierte und unsubstituierte Reste mit 4 bis 8 Kohlenstoffatomen in Betracht.
Als Arylreste R³ und R⁴ kommen substituierte und unsubstituierte carbocyclische oder heterocyclische Reste wie z.B. Phenyl, Naphtyl, Furyl, Thienyl, Pyridyl, Pyrazolyl, Pyrimidinyl, Oxazolyl, Pyridazinyl, Pyrazinyl, Chinolyl, die mehrfach substituiert sein können durch Halogen, OH, C₁-C₂₀-Alkoxy, CO₂H, CO₂-Alkyl, -NO₂, -N₃, -CN, C₁-C₂₀-Alkyl, C₁-C₂₀-Acyl, C₁-C₂₀-Acyloxy-Gruppen, in Frage.
Die in R¹, R², und R⁴ der allgemeinen Formel I enthaltenen Alkanoylgruppen sollen jeweils 1 bis 20 Kohlenstoffatome enthalten, wobei Formyl, Acetyl, Propionyl- und Isopropionylgruppen bevorzugt sind.
Die Aralkylgruppen in R¹, R², R³ und R⁴ können im Ring bis 14 C-Atome, bevorzugt 6 bis 10 und in der Alkylkette 1 bis 8, bevorzugt 1 bis 4 Atome enthalten. Als Aralkylreste kommen beispielweise in Betracht Benzyl, Phenylethyl, Naphtylmethyl, Naphtylethyl, Furylmethyl, Thienylethyl, Pyridylpropyl. Die Ringe können einfach oder mehrfach substituiert sein durch Halogen, OH, O-Alkyl, CO₂H, CO₂-Alkyl, -NO₂, -N₃, -CN, C₁-C₂₀-Alkyl, C₁-C₂₀-Acyl, C₁-C₂₀-Acyloxy-Gruppen.
Als Aroylreste für R¹, R² und R⁴ sind Benzoate sowie im Phenylrest substituierte Benzoate zu bevorzugen.
Eine Alkenylen- bzw. Alkinylengruppe als E enthält typischerweise 1 bis 3 Unsättigungen.
Freie Hydroxygruppen in I können funktionell abgewande. sein, beispielsweise durch Veretherung oder Veresterung; freie Hydroxygruppen sind jedoch bevorzugt.
Als Ether-, Acylreste und Schutzgruppe PG kommen die dem Fachmann bekannten Reste wie z.B. der Methoxymethyl-, Methoxyethyl-, Ethoxyethyl-, Tetrahydropyranyl-, Tetrahydrofuranyl-, Trimethylsilyl-. Triethylsilyl-, tert.-Butyldimethylsilyl-, tert.-Butyldiphenylsilyl-, Tribenzylsilyl-, Triisopropylsilyl-, Methyl-, tert.-Butyl-, Benzyl-, para-Nitrobenzyl-para-Methoxybenzyl, Formyl-, Acetyl-, Propionyl-, Isopropionyl-, Butyryl-, Pivaloyl, Benzoyl-Rest in Frage. Eine Übersicht befindet sich z.B. in "Protective Groups in Organic Synthesis" Theodora W. Green, John Wiley and Sons).
Als spezifische Seitenketten, in denen X für ein Sauerstoffatom steht, seien genannt

-O-(CH₂)₅S(CH₂)₃C₂F₅

-O-(CH₂)₅SO(CH₂)₃C₂F₅

-O-(CH₂)₅SO₂(CH₂)₃C₂F₅

-O-(CH₂)₄S(CH₂)₃C₂F₅

-O-(CH₂)₄SO(CH₂)₃C₂F₅

-O-(CH₂)₄SO₂(CH₂)₃C₂F₅

-O-(CH₂)₅-Cl

-O-(CH₂)₄-Cl

-O-(CH₂)₃-Cl

-O-(CH₂)₂-Cl

-O-(CH₂)₂-N(CH₃)₂

-O-(CH₂)₂-1-Pyrrolidinyl

Als Seitenketten, worin X für eine direkte Bindung steht, kommen beispielsweise in Betracht (DE 1 98 06 357.1)

-(CH₂)₅N(CH₃)(Ch₂)₃C₂F₅

-(CH₂)₅N(CH₃)(CH₂)₆C₂F₅

-(CH₂)₅N(CH₃)(CH₂)₇C₂F₅

-(CH₂)₅N(CH₃)(CH₂)₈C₂F₅

-(CH₂)₆N(CH₃)(CH₂)₆C₂F₅

-(CH₂)₆N(CH₃)(CH₂)₇C₂F₅

-(CH₂)₆N(CH₃)(CH₂)₈C₂F₅

-(CH₂)₅N(CH₃)(CH₂)₂C₄F₉

-(CH₂)₅N(CH₃)(CH₂)₃C₆F₁₃

-(CH₂)₅N(CH₃)(CH₂)₃C₈F₁₇

-(CH₂)₅N(CH₃)(CF₂)₆C₄F₉

-(CH₂)₅N(CH₃)(CH₂)₆C₆F₁₃

-(CH₂)₅N(CH₃)(CH₂)₆C₈F₁₇

-(CH₂)₅N(CH₃)H

-(CH₂)₅N(CH₃)(CH₂)₉H

-(CH₂)₅-1-Pyrrolidinyl

-(CH₂)₅N(CH₃)(CH₂)₃OPhenyl

-(CH₂)₅N(CH₃)(CH₂)₃OBenzyl

-(CH₂)₅N(CH₃)(CH₂)₃O(CH₂)₃C₂F₅

-(CH₂)₉S(CH₂)₃C₂F₅

-(CH₂)₉SO(CH₂)₃C₂F₅

-(CH₂)₉SO₂(CH₂)₃C₂F₅

Desweiteren kommen in Betracht die Seitenketten der allgemeinen Teilformel 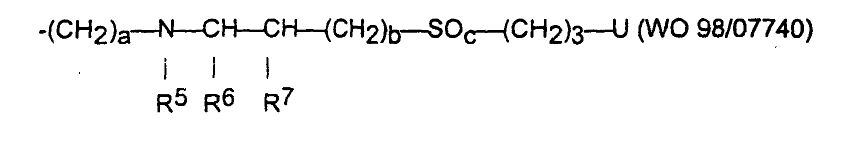 wobei
a 4, 5 oder 6,
b 0, 1 oder 2,
c 0, 1 oder 2,
R⁵ ein Wasserstoffatom oder eine C₁₋₅-Alkylgruppe,
R⁶ und R⁷ je ein Wasserstoffatom, oder
R⁵ und R⁶ gemeinsam eine Alkylengruppe ―(CH₂)_{d}― mit d = 2, 3, 4 oder 5 und R⁷ ein Wasserstoffatom oder
R⁵ und R⁷ gemeinsam eine Alkylengruppe ―(CH₂)ₑ― mit e = 2, 3 oder 4 und R⁶ ein Wasserstoffatom, und
U ein unsubstituierter oder ein- bis fünffach fluorierter Ethylrest ist, oder der terminale Substituent -(CH₂)₃-U in der Seitenkette durch einen gegebenenfalls substituierten Aryl- oder Heteroarylrest, der direkt oder über eine Mono-, Di- oder Trimethylengruppe an das Schwefelatom gebunden ist, ersetzt ist,
und von diesen wiederum insbesondere die Seitenketten

-(CH₂)₅N(CH₃)(CH₂)₃S(CH₂)₃C₂F₅

und

-(CH₂)₅N(R⁵)(CHR⁶)CH₂S(CH₂)₃C₂F₅ mit R⁵+R⁶ = -(CH₂)₃-.

Spezifische Verbindungen der allgemeinen Formel l sind in den Beispielen beschrieben.

Die vorliegende Erfindung betrifft außer diesen Verbindungen der allgemeinen Formel l, wenn in Y ein Stickstoffatom enthalten ist, auch deren physiologisch verträgliche Additionssalze mit organischen und anorganischen Säuren, diese Verbindungen der allgemeinen Formel l inclusive der Additionssalze enthaltende pharmazeutische Präparate sowie deren Verwendung zur Herstellung von Arzneimitteln.

Zur Bildung von Säureadditionssalzen sind anorganische und organische Säuren geeignet, wie sie dem Fachmann zur Bildung physiologisch verträglicher Salze bekannt sind. Als Additionssalze mit Säuren sind insbesondere die Hydrochloride, Hydrobromide, Acetate, Citrate, Oxalate, Tartrate und die Methansulfonate zu nennen.

Die Verbindungen der allgemeinen Formel I stellen Verbindungen mit starker antiestrogener Wirksamkeit dar.

Bei den erfindungsgemäßen Verbindungen handelt es sich zum einen Teil um reine Antiestrogene oder zum anderen Teil um sogenannte Partialantagonisten, d. h. um Antiestrogene mit estrogener Partialwirkung wie das Tamoxifen oder das Raloxifen. Im Gegensatz zum Tamoxifen tritt bei den Partialantagonisten der allgemeinen Formel I deren agonistische, estrogene Wirkung gewebeselektiv auf. Insbesondere tritt die agonistische Wirkung am Knochen, im Herz-Kreislaufsystem und im ZNS (Zentrales Nervensystem) auf. Insbesondere tritt am Uterus keine oder nur geringe agonistische Wirkung auf.

Verbindungen mit antiestrogenen Eigenschaften, d.h. Stoffe mit Hemmwirkungen gegenüber Estrogenen, sind bereits zahlreich beschrieben worden.
Antiestrogen wirksame Verbindungen mit einem den vorliegenden Verbindungen vergleichbaren 3,4-Diphenyl-bicyclo [4.3.0]nonyl-Grundgerüst existieren bisher aber nicht.

Das erstmals aus BE 637,389 hervorgehende Tamoxifen, (Z)-2-[4-(1,2-Diphenyl-1 -butenyl)-phenoxy]-N,N-dimethylethylamin, wird seit längerem als Antiestrogen zur Therapie von Brustkrebs eingesetzt.

Das aus der EP A 0 138 504 B1 hervorgehende Steroid-Derivat 7α-[9-(4,4,5,5,5-Pentafluorpentylsulfinyl)-n-nonyl]-estra-1,3,5(10)-trien-3,17β-diol (EP A 0 138 504, Seite 58, vorletzte Verbindung) befindet sich gegenwärtig in klinischer Entwicklung für hormonabhängige Tumoren (Brustkrebs).
Pharmazeutische Zusammensetzungen, die Sexualsteroid-Inhibitoren enthalten, welche ein steroidales Grundgerüst, das eine 7α-Seitenkette bei gleichzeitiger Anwesenheit mindestens eines weiteren Substituenten in Position 14, 15 oder 16 aufweist, sind Gegenstand der EP-A 0 376 576.

Eine Vielzahl verschiedenartigster Verbindungen - u. a. solche steroidalen Ursprungs als auch solche mit 2-Phenylindol-Grundgerüst - die als Antiestrogen wirken und/oder die Estrogenbiosynthese unterdrücken, werden in der WO 93/10741 offenbart.

Weitere steroidale Antiestrogene, die einen 11β-Phenylrest tragen, sind in den EP-AS 0 384 842 und 0 629 635 beschrieben.

Der Uteruswachstumstest bei der infantilen Ratte, p.o. (Test auf antiestrogene Wirkung in-vivo) belegt die antiestrogene Wirksamkeit der erfindungsgemäßen Verbindungen. Der Test läßt sich wie nachstehend beschrieben durchführen:

### Uteruswachstumstest bei der infantilen Ratte (antiestrogene Wirkung)

### Prinzip der Methode

Bei Nagern reagiert der Uterus auf die Applikation von estrogenen mit einer Gewichtszunahme (sowohl Proliferation als auch Wassereinlagerung). Dieses Wachstum ist durch gleichzeitige Gabe antiestrogen-wirkender Verbindungen dosisabhängig zu hemmen.

### Versuchsdurchführung

### Tiere:

Infantile weibliche Ratten im Gewicht von 35-45 g bei Versuchsbeginn, pro Dosis 5-6 Tiere.

### Formulierung und Applikation der Substanzen:

Für die p.o. Applikation werden die Substanzen in 1 Teil Ethanol (E) gelöst und mit 9 Teilen Erdnußöl (EÖ) aufgefüllt.

### Versuchsansatz

Die gerade von den Müttern abgesetzten jungen Ratten werden zur Eingewöhnung einen Tag vor Behandlungsbeginn geliefert und sofort mit Futter - auch in dem Tierkäfig - versorgt.
Die Behandlung erfolgt dann täglich einmal über 3 Tage in Kombination mit 0,5 µg Estradiolbenzoat (ES). ES wird immer subcutan (s.c.) appliziert. während die Testsubstanz p.o. (peroral) verabreicht wird. 24 Stunden nach der letzten Applikation werden die Tiere gewogen, getötet und die Uteri entnommen. Von den präparierten Uteri werden die Feuchtgewichte (ohne Inhalt) ermittelt.

### Kontrollen

negative Kontrolle: Vehikel (E/EÖ). 0,2 ml/Tier/Tag positive Kontrolle: 0,5 µg EB/0.1 ml/Tier/Tag

### Auswertung

Von den relativen Organgewichten (mg/100 g Körpergewicht) werden für jede Gruppe die Mittelwerte mit Standardabweichung (X+SD), sowie die Signifikanz der Unterschiede zur Kontrollgruppe (EB) im Dunnett-Test (p<0.05) ermittelt.
Die Berechnung der Hemmung (in %) gegenüber der EB-Kontrolle erfolgt mit einem Programm. Die relativen Wirksamkeiten der Prüfsubstanzen werden durch eine Kovarianz- und Regressionsanalyse ermittelt.

| **Antiuterotrophe Wirkung an der Ratte** | |
|---|---|
| Verbindung aus Beispiel | Antiuterotrophe Wirkung bei 0,3 mg s.c. [% Hemmung] |
| 5 | 55 |
| 3 | 37 |

Die erfindungsgemäßen Verbindungen, insbesondere wenn es reine Antiestrogene sind, eignen sich zur Therapie von estrogen-abhängigen Erkrankungen, zum Beispiel Mammacarcinom (second-line Therapie des Tamoxifen-resistenten Mammacarcinoms; zur adjuvanten Behandlung des Mammacarcinoms anstelle von Tamoxifen), Endometriumcarcinom, Prostatacarcinom, Prostatahyperplasie, anovulatorische Infertifität und Melanom.
Die reinen Antiestrogene der allgemeinen Formel I können außerdem als Komponente in den in der EP 346 014 B1 beschriebenen Produkten verwendet werden, die ein Estrogen und ein reines Antiestrogen enthalten, und zwar zur gleichzeitigen, sequentiellen oder getrennten Verwendung für die selektive Estrogentherapie peri- oder postmenopausaler Frauen.
Die Verbindungen der allgemeinen Formel I, insbesondere wenn es sich um reine Antiestrogene handelt, können gemeinsam mit Antigestagehen (kompetitiven Progesteronantagonisten) zur Behandlung hormonabhängiger Tumoren verwendet werden (EP 310 542 A).
Weitere Indikationen, in denen die Verbindungen der allgemeinen Formel zum Einsatz kommen können, ist der männliche Haarausfall, eine diffuse Alopecie, eine durch eine Chemotherapie hervorgerufene Alopecie sowie Hirsutismus (Hye-Sun Oh und Robert C. Smart, Proc. Natl. Acad. Sci. USA, 93 (1996) 12525 - 12530).
Außerdem können die Verbindungen der allgemeinen Formel l zur Herstellung von Medikamenten zur Behandlung der Endometriose und von Endometrialkarzinomen verwendet werden.
Ferner kann man die Verbindungen der allgemeinen Formel l zur Herstellung pharmazeutischer Zusammensetzungen für die männliche und weibliche Fertilitätskontrolle einsetzen (männliche Fertilitätskontrolle: DE-A 195 10 862.0).

Diejenigen Verbindungen der allgemeinen Formel mit gewebeselektiver estrogener Partialwirkung können in erster Linie zur Prophylaxe und Therapie der Osteoporose und zur Herstellung von Präparaten für die Substitutionstherapie in der Prae-, Peri- und Postmenopause (HRT = Hormone Replacement Therapy; Hormonersatz-Therapie) Verwendung finden (Black, L.J., Sato,M.,Rowley, E.R.,Magee, D.E., Bekele, A., Williams, D.C., Cullinan, G.J., Bendele, R., Kauffman. R.F., Bensch, W.R., Frolik, C.A., Termine, J.D. and Bryant, H.U.: Raloxifene [LY 139481 HCI] prevents bone loss and reduces serum cholesterol without causing uterine hypertrophy in ovariectomized rats; J. Clin. Invest. 93: 63 - 69, 1994).

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere zur Behandlung von estrogenabhängigen Krankheiten und Tumoren und von Arzneimitteln für die Hormonsubstitutions-Therapie (HRT).

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u. ff.; H.v. Czetsch-Lindenwald, Hilfsstoffe für Pharmazie und angrenzende Gebiete; Pharm. Ind. Heft 2, 1961, Seite 72 u. ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan oder perkutan, verabreicht werden. Die Verbindungen können auch in das Gewebe implantiert werden. Die zu verabreichende Menge der Verbindungen schwankt innerhalb eines weiten Bereichs und kann jede wirksame Menge abdecken. In Abhängigkeit des zu behandelnden Zustandes und der Art der Verabreichung kann die Menge der verabreichten Verbindung 0,1-25 mg/kg Körpergewicht, vorzugsweise 0,5-5 mg/kg Körpergewicht, je Tag betragen. Beim Menschen entspricht dies einer täglichen Dosis von 5 bis 1250 mg.
Die bevorzugte tägliche Dosierung beim Menschen ist 50 bis 200 mg. Dies gilt insbesondere für die Tumortherapie.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 5 bis 500 mg des Wirkstoffs enthalten.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Erfindungsgemäß können die Verbindungen der allgemeinen Formel I auch mit Liposomen verkapselt werden.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittfers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen der allgemeinen Formel l können auch in Form einer Lösung formuliert werden, die für die orale Verabreichung bestimmt ist und die neben der aktiven Verbindung der allgemeinen Formel enthält
a) ein pharmazeutisch verträgliches Öl und/oder
b) eine pharmazeutisch verträgliche lipophile oberflächenaktive Substanz und/oder
c) eine pharmazeutisch verträgliche hydrophile oberfächenaktive Substanz und/oder
d) ein pharmazeutisch verträgliches wassermischbares Lösungsmittel.
Hierzu wird außerdem auf die WO 97/21440 verwiesen.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder intrauterinsystemen (z.B. Pessare, Spiralen) eignen sich verschiedene Polymere wie zum Beispiel Silikonpolymere. Ethylenvinylacetat, Polyethylen oder Polypropylen.

Die erfindungsgemäßen Verbindungen können alleine oder zur Erzielung additiver oder synergistischer Wirkungen in Kombination mit weiteren in der Tumortherapie anwendbaren Prinzipien und Substanzklassen verwendet werden.
Als Beispiele seien genannt die Kombination mit
○ Platinkomplexen wie z.B. Cisptatin, Carboplatin,
○ interkalierenden Substanzen z.B. aus der Klasse der Anthracycline wie z.B. Doxorubicin oder aus der Klasse der Anthrapyrazole wie z.B. Cl-941,
○ mit Tubulin interagierenden Substanzen z.B. aus der Klasse der Vinka-Alkaloide wie z.B. Vincristin, Vinblastin oder aus der Klasse der Taxane wie z.B. Taxol, Taxotere oder aus der Klasse der Makrolide wie z.B. Rhizoxin und seinen Analoga, Epothilon B und seinen Analoga, Discodermolid und seinen Analoga, Eleutherobin und seinen Analoga oder andere Verbindungen wie z.B. Colchicin, Combretastatin A-4,
○ DNA Topoisomeraseinhibitoren wie z.B. Camptothecin, Etoposid, Topotecan, Teniposid,
○ Folat- oder Pyrimidin-Antimetaboliten wie z.B. Lometrexol, Gemcitubin,
○ DNA alkylierenden Verbindungen wie z.B. Adozelesin, Distamycin A,
○ Inhibitoren von Wachstumsfaktoren (z.B. von PDGF, TGFβ, EGF) wie z.B. Somatostatin, Suramin, Bombesin-Antagonisten,
○ Inhibitoren der Protein Tyrosin Kinase oder der Protein Kinasen A oder C wie z.B. Erbstatin, Genistein, Staurosporin, limofosin, 8-Cl-cAMP,
○ Antihormonen aus der Klasse der Antigestagene wie z.B. Mifepriston, Onapriston oder aus der Klasse der Antiöstrogene wie z.B. Tamoxifen oder aus der Klasse der Antiandrogene wie z.B. Cyproteronacetat (Kombination mit Antigestagenen siehe beispielsweise EP 0 310 542 B1 und EP 0 310 541 B1),
○ Metastasen inhibierenden Verbindungen z.B. aus der Klasse der Eicosanoide wie z.B. PGl₂, PGE₁, 6-Oxo-PGE₁ sowie deren stabiler Derivate (z.B. Iloprost, Cicaprost),
○ Inhibitoren onkogener RAS-Proteine, welche die mitotische Signaltransduktion beeinflussen, wie beispielsweise Inhibitoren der Farnesyl-Protein-Transferase,
○ natürlichen oder künstlich erzeugten Antikörpern, die gegen Faktoren bzw. deren Rezeptoren, die das Tumotwachstum fördern, gerichtet sind, wie beispielsweise der erbB2-Antikörper.

Die Erfindung betrifft ferner Verfahren zur Herstellung von Verbindungen der Formel I, die im folgenden Schema 1 näher beschrieben werden.

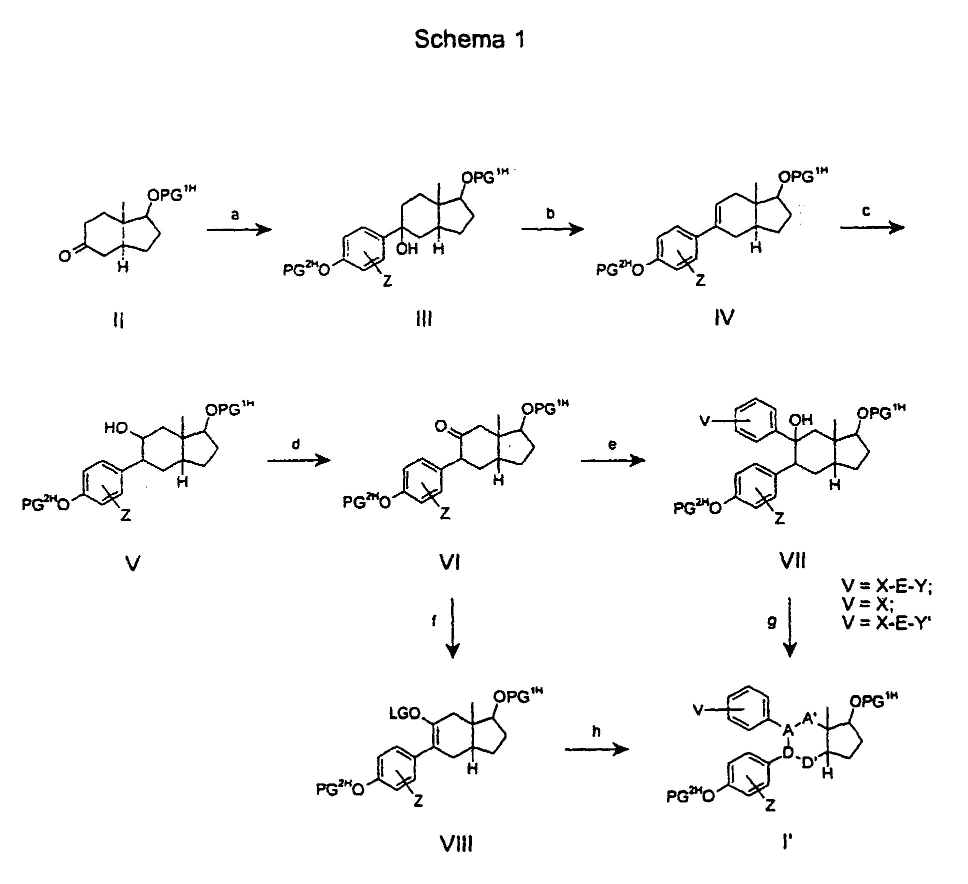

### Schritt a (II ⇒ III):

Das Keton II , worin PG ^{1H} die oben für PG ¹ genannten Bedeutungen haben kann oder ein Wasserstofatom bedeutet, wird nach literaturbekannten Verfahren hergestellt. Die Umsetzung zu einer Arylverbindung der Formel III erfolgt durch Reaktion mit einer metallorganischen Verbindung der allgemeinen Formel 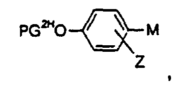 worin M für ein Alkalimetall oder für ein zweiwertiges Metallatom M-Hal, worin Hal ein Halogenatom ist, steht, PG^{2H} die oben für PG² und Z die oben genannten Bedeutungen aufweisen oder PG^{2H} ein Wasserstoffatom bedeutet und freie OH-, CO₂H oder NH₂-Gruppen in Z gegebenenfalls geschützt sind. Als zweiwertiges Metall ist bevorzugt Magnesium und Zink, als Halogen Hal ist bevorzugt Chlor, Brom und Iod. Die Umsetzung erfolgt in einem inerten Lösungsmittel, vorzugsweise in Tetrahydrofuran oder Diethylether. Als PG^{1H} ist Wasserstoff oder eine Schutzgruppe PG¹ in der Bedeutung von tert.-Butyl- oder tert.-Butyldimethylsilyl, als PG^{2H} ist Wasserstoff oder eine Schutzgruppe PG² in der Bedeutung von Methyl-, Benzyl- oder tert.-Butyldimethylsilyl bevorzugt. Eine selektive Modifikation der Schutzgruppen PG¹ und/oder PG² ist möglich.

### Schritt b (III => IV):

Die Eliminierung von Wasser in III zum Olefin IV erfolgt nach den, dem Fachmann bekannten Methoden. Bevorzugt ist die Verwendung mineralischer wässriger Säuren und inerten organischen, mit Wasser mischbaren Lösungsmitteln wie beispielsweise Dioxan oder Tetrahydrofuran. Unter diesen Bedingungen werden ebenfalls sauer spaltbare Schutzgruppen PG¹ und/oder PG² eliminiert. Eine selektive Modifikation der Schutzgruppen PG¹ und/oder PG² ist möglich. Als PG^{1H} ist Wasserstoff oder eine Schutzgruppe PG¹ in der Bedeutung von tert.-Butyl- oder tert.-Butyldimethylsilyl bevorzugt, als PG^{2H} ist Wasserstoff oder eine Schutzgruppe PG² in der Bedeutung von Benzyl- oder tert.-Butyldimethylsityl bevorzugt.

### Schritt c (IV => V):

An die Doppelbindung in IV wird nach anti-Markovnikov Wasser addiert. Hierzu eignen sich die dem Fachmann bekannten Verfahren wie z.B. die Umsetzung mit Boranen, deren anschließende Oxidation zu den entsprechenden Borsäureestern und deren Verseifung. Als Borane bevorzugt sind z.B. der Boran-Tetrahydrofuran-Komplex, der Boran-Dimethylsulfid-Komplex, 9-Borabicyclo[3.3.1]nonan in einem inerten Lösungsmittel wie beispielsweise Tetrahydrofuran oder Diethylether. Als Oxidationsmittel wird vorzugsweise Wasserstoffperoxid verwendet, zur Verseifung der Borester vorzugsweise Alkalihydroxide wie z.B. Natriumhydroxid.

### Schritt d (V⇒ VI):

Die Oxidation des Alkohols in V zum Keton erfolgt nach den, dem Fachmann bekannten Methoden. Beispielsweise genannt sei die Oxidation mit Pyridiniumchlorochromat, Pyridiniumdichromat, Chromtrioxid-Pyridin-Kompfex, die Oxidation nach Swern oder verwandter Methoden z.B. unter Verwendung von Oxalylchlorid in Dimethylsulfoxid, die Verwendung des Dess-Martin-Periodinans, die Verwendung von Stickstoffoxiden wie z.B. N-Methylmorpholino-N-oxid in Gegenwart geeigneter Katalysatoren wie z.B. Tetrapropylammoniumperruthenat in inerten Lösungsmitteln. Bevorzugt ist die Oxidation mit dem Chromtrioxid-Pyridin-Komplex. Eine selektive Modifikation der Schutzgruppen PG¹ und/oder PG² ist möglich. Als PG^{1H} ist Wasserstoff oder eine Schutzgruppe PG¹ in der Bedeutung Tetrahydropyranyl oder tert.-Butyldimethylsilyl bevorzugt, als PG^{2H} ist Wasserstoff oder eine Schutzgruppe PG² in der Bedeutung von. Tetrahydropyranyl, Benzyl- oder tert.-Butyldimethylsilyl bevorzugt.

### Schritt e (VI ⇒ VII):

Die Umsetzung des Ketons VI zu einer Arylverbindung der Formel VII erfolgt durch Reaktion mit einer metallorganischen Verbindung der allgemeinen Formel 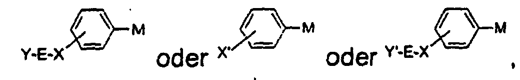 worin M für ein Alkalimetall oder für ein zweiwertiges Metallatom M-Hal, worin Hal ein Halogenatom ist, steht und Y, E, X die oben genannten Bedeutungen haben, freie OH-, SH- oder NH-Gruppen in Y gegebenenfalls geschützt sind, X' für eine geschützte Hydroxygruppe OPG und Y' für eine Gruppe OLG, ein Halogenatom (F, Cl, Br, I) oder eine Gruppe OPG stehen. Als zweiwertiges Metall ist bevorzugt Magnesium und Zink, als Halogen Hal ist bevorzugt Chlor, Brom und Iod. Die Umsetzung erfolgt in einem inerten Lösungsmittel, vorzugsweise in Tetrahydrofuran oder Diethylether. Eine selektive Modifikation von Y sowie der Schutzgruppen PG¹ und/oder PG² ist möglich. Als PG^{1H} ist Wasserstoff oder eine Schutzgruppe PG¹ in der Bedeutung Acetyl-, Tetrahydropyranyl oder tert.-Butyldimethylsifyl, als PG^{2H} ist Wasserstoff oder eine Schutzgruppe PG² in der Bedeutung Tetrahydropyranyl, Benzyl- oder tert.-Butyldimethylsilyl bevorzugt.

In X' bzw. Y' freigesetzte Hydroxylgruppen können durch Veretherung weiter modifiziert werden.
Stellt Y' ein Halogen, vorzugsweise Cl, Br, I oder eine Abgangsgruppe OLG dar, so kann der weitere Kettenaufbau beispielsweise durch Alkylierung, Aminierung (z.B. mit HNR^{4a}R^{4b}, HNR^{4a}(CH₂)ₚ-Q-G) oder Veretherung (z.B. mit HO-G, HS-G) erfolgen.
Zum Aufbau der Seitenkette wird im übrigen auf die in EP A 0 138 504, EP-A 0 376 576, WO 98/07740 und der DE 1 98 06 357.1 dort für den Aufbau der 7α-Seitenkette des Steroids beschriebenen Verfahren und Beispiele verwiesen. Durch analoge Vorgehensweise lassen sich die dort beschriebenen und syntethisierten Seitenketten in den vorliegenden Verbindungen als -X-E-Y aufbauen.

### Schritt f (VI ⇒VIII):

Die Umsetzung des Ketons VI zu einer Enolverbindung der Formel VIII, worin LG eine Abgangsgruppe darstellt, erfolgt nach den, dem Fachmann bekannten Verfahren. Durch Anwendung einer starken Base in einem inerten Lösungsmittel wie beispielsweise Tetrahydrofuran oder Diethylether wird das Enolat des Ketons VI erzeugt und anschließend mit einer Verbindung LG-Hal, worin Hal die Bedeutung Fluor, Chlor, Brom oder Iod besitzt, umgesetzt. Als Abgangsgruppe LG kommen beispielsweise Alkylsulfonyle oder gegebenenfalls substituierte Arylsulfonyle, beispielsweise der Tosyfatrest, in Frage; bevorzugt sind perfluorierte Alkylsulfonyle wie beispielsweise Perfluorbutylsulfonyl oder Trifluormethylsulfonyl. Als PG^{1H} ist eine Schutzgruppe PG¹ in der Bedeutung von Tetrahydropyranyl oder tert.-Butyldimethylsilyl, als PG^{2H} eine Schutzgruppe PG² in der Bedeutung von Tetrahydropyranyl, Benzyl- oder tert.-Butyldimethylsilyl bevorzugt.

### Schritt g (VII ⇒ I'):

Die Eliminierung von Wasser in VII zu Verbindungen der Formel I' mit A-D in der Bedeutung einer C-C-Doppelbindung erfolgt nach den unter Schritt b genannten Bedingungen. Die Doppelbindung läßt sich nach den, dem Fachmann bekannten Verfahren gegebenenfalls hydrieren (A-D in der Bedeutung einer C-C-Einfachbindung) oder oxidieren (A-D in der Bedeutung einer 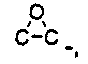 einer COH-CH-, einer CH-COH- oder einer COH-COH-Gruppe). Eine selektive Modifikation von Y sowie der Schutzgruppen PG¹ und/oder PG² ist möglich. Als PG ^{1H} ist Wasserstoff oder eine Schutzgruppe PG¹ in der Bedeutung Acetyl-, Tetrahydropyranyl oder tert.-Butyldimethylsilyl, als PG^{2H} ist Wasserstoff oder eine Schutzgruppe PG² in der Bedeutung Tetrahydropyranyl, Benzyl- oder tert.-Butyldimethylsilyl bevorzugt.

### Schritt h (VIII ⇒ I'):

Die Umsetzung der Verbindung VIII erfolgt nach den, dem Fachmann bekannten Methoden. Vorzugsweise wird mit einer Boronsäure der Formel 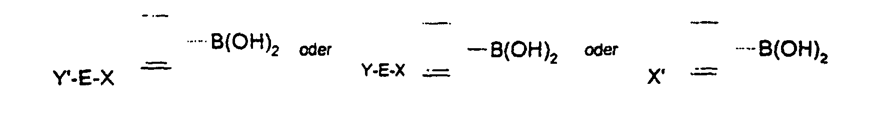 worin Y, E, X, Y' und X' die oben genannten Bedeutungen haben, freie OH-, SH- oder NH-Gruppen in Y gegebenenfalls geschützt sind, unter Palladium(0)-Katalyse zu einer Verbindung der Formel mit A-D in der Bedeutung einer C-C-Doppelbindung umgesetzt. Die Doppelbindung läßt sich nach den, dem Fachmann bekannten Verfahren gegebenenfalls hydrieren (A-D in der Bedeutung einer C-C-Einfachbindung) oder oxidieren (A-D in der Bedeutung
Bedeutung einer 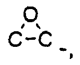 einer COH-CH-, einer CH-COH- oder einer COH-COH-Gruppe). Eine selektive Modifikation von Y sowie der Schutzgruppen PG¹ und/oder PG² ist möglich. Als PG^{1H} ist Wasserstoff oder eine Schutzgruppe PG¹ in der Bedeutung Acetyl-, Tetrahydropyranyl oder tert.-Butyldimethylsilyl, als PG^{2H} ist Wasserstoff oder eine Schutzgruppe PG² in der Bedeutung Tetrahydropyranyl, Benzyl- oder tert.-Butyldimethylsilyl bevorzugt. Die Spaltung gegebenenfalls in l' enthaltener Schutzgruppen nach den, dem fachmann bekannten Verfahren führt zu den erfindungsgemäßen Verbindungen l.

Die erfindungsgemäßen Verbindungen können wie nachstehend beschrieben hergestellt werden. Durch analoge Vorgehensweise unter Verwendung analoger Reagenzien zu den in den Beispielen enthaltenen Angaben lassen sich weitere Verbindungen der allgemeinen Formel I erhalten.

Als Verfahren zum Aufbau der Seitenkette -X-E-Y in den erfindungsgemäßen Verbindungen sind insbesondere auch die in der EP 0 138 504 B1 und der EP 0 376 576 A beschriebenen Methoden der Seitenketteneinführung und des Seitenkettenaufbaus geeignet.

Eine Thiobrücke in der Seitenkette kann mit Natriumperiodat zum Sulfoxid oxidiert werden; mit einer Persäure als Oxidationsmittel, z.B. *m*-Chlorperbenzoesäure, werden aus den Sulfiden die Sulfoxide und Sulfone erhalten.

Die Verseifung von Estergruppierungen sowieVeresterung und Veretherung freier Hydroxygruppen erfolgt jeweils nach etablierten Verfahren der organischen Chemie.

Die Säureadditionssalze der Verbindungen der allgemeinen Formel I lassen sich ebenfalls nach gängigen Verfahren aus den Verbindungen der allgemeinen Formel I herstellen.

Die nachfolgenden Beispiele dienen der näheren Erläuterung der Erfindung:

### Beispiel 1

### (1S,3S,4R,6S,9S)-9-Hydroxy-4-(4-hydroxyphenyl)-1-methyl-3-[4-(10,10,11,11,11-pentafluor-6-thia-undecyloxy)phenyl]-bicyclo[4.3.0]nonan

### Beispiel 1a

### (1S,4R,6S,9S)-4-(4-Methoxyphenyl)-4-hydroxy-1-methyl-9-tert.-butyloxy-bicyclo [4.3.0]nonan (A) und (1S,4S,6S,9S)-4-(4-Methoxyphenyl)-4-hydroxy-1-methyl-9-tert.-butytoxy-bicyclo[4.3.0]nonan (B)

Unter einer trockenen Atmosphäre aus Argon wird die Suspension aus 4,8 g Magnesiumspäne in 40 ml wasserfreiem Tetrahydrofuran mit 0,2 ml 1,2-Dibrommethan versetzt, 24,6 ml 4-Bromanisol in 260 ml Tetrahydrofuran so langsam zugetropft, daß die Innentemperatur 28°C nicht überschreitet und noch 1 Stunde bei 23°C nachgerührt. Anschließend versetzt man mit der Lösung von 10,0 g (44,6 mmol) (1S,6S,9S)-9-tert.-Butyloxy-1-methyl-bicyclo[4.3.0)nonan-4-on in 100 ml Tetrahydrofuran und rührt 16 Stunden bei 23°C. Bei 0°C wird mit gesättigter Ammoniumchloridlösung versetzt, mit Wasser und Ethylacetat verdünnt, die organische Phase abgetrennt, mit gesättigter Natriumchloridlösung gewaschen und über Natriumsulfat getrocknet. Den nach Filtration und Lösungsmittelabzug erhaltenen Rückstand reinigt man durch Chromatographie an ca. 1,5 l feinem Kieselgel mit einem Gradientensystem aus n-Hexan und Ethylacetat. Isoliert werden 5,45 g (16,4 mmol, 37%) der Titelverbindung A sowie 8,16 g (24,5 mmol, 55%) der Titelverbindung B jeweils als farbloser Schaum.
¹H-NMR (CDCl₃) von A: δ = 0,82 (3H), 1,16 (9H), 1,30-1,73 (8H), 1,78-2,06 (4H), 3,53 (1H), 3,80 (3H), 6,88 (2H), 7,43 (2H) ppm.
¹H-NMR (CDCl₃) von B: δ = 0,90 (3H), 1,08 (9H), 1,13 (1H), 1,28-1.60 (4H), 1,68-1,89 (4H). 2,00 (1H), 2,22 (1H), 2,35 (1H), 3,26 (1H), 3,82 (3H), 6,91 (2H), 7,50 (2H) ppm.

### Beispiel 1b

### (1S,4S,6S,9S)-4-(4-Hydroxyphenyl)-9-tert.-butyloxy-4-hydroxy-1-methyl-bicyclo[4.3.0]nonan

In Analogie zu Beispiel 1c werden 8,16 g (24,5 mmol) der nach Beispiel 1a dargestellten Verbindung B umgesetzt und das nach Aufarbeitung erhaltene Rohprodukt zusammen mit dem in Beispiel 1c erhaltenen Rohprodukt aufgereinigt.

### Beispiel 1c

### (1S,6S,9S)-4-(4-Hydroxyphenyl)-9-tert.-butyloxy-1-methyl-bicyclo[4.3.0]non-3-en (A) und (1S,4R,6S,9S)-4-(4-Hydroxyphenyl)-9-tert.-butyloxy-4-hydroxy-1-methyl-bicyclo[4.3.0]nonan (B)

Die Lösung von 5,45 g (16,4 mmol) der nach Beispiel 1a dargestellten Verbindung A in 70 ml wasserfreiem Dimethylformamid versetzt man unter einer Atmosphäre aus trockenem Argon mit 5,65 g Natriummethanthiolat und erhitzt ca. 5 Stunden auf 170°C. Nach dem Erkalten gießt man in Wasser extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand vereinigt man mit dem in Beispiel 1b erhaltenen Rohprodukt und reinigt durch Chromatographie an ca. 800 ml feinem Kieselgel mit einem Gradientensystem aus n-Hexan und Ethylacetat. Isoliert werden 8,00 g (26,6 mmol, 65% bezogen auf die in Beispiel 1a erhaltenen Produkte A und B) der Titelverbindung A sowie 1,85 g (5,81 mmol, 14% bezogen auf die in Beispiel 1a erhaltenen Produkte A und B) der Titelverbindung B jeweils als farbloser Schaum.
¹H-NMR (CDCl₃+CD₃OD) von A: δ = 0,72 (3H), 1,14 (9H), 1,33-1,74 (4H), 1,83-2,03 (2H), 2,03-2,21 (2H), 2,26-2,54 (2H), 3,52 (1H), 5,91 (1H), 6,72 (2H), 7,23 (2H) ppm.
¹H-NMR (CDCl₃+CD₃OD) von B: δ = 0,78 (3H), 1,14 (9H), 1,20-2,01 (13H), 3,51 (1H). 6,76 (2H), 7,30 (2H) ppm.

### Beispiel 1d

### (1S,6S,9S )-4-(4-Benzyloxyphenyl)-9-tert.-butyloxy-1-methyl-bicyclo[4.3.0]non-3-en und

### (1S,4R,6S,9S)-4-(4-Benzyloxyphenyl)-9-tert.-butyloxy-4-hydroxy-1-methyl-bicyclo[4.3.0]nonan

1,85 g (5,81 mmol) der nach Beispiel 1c dargestellten Verbindung B behandelt man in Analogie zu Beispiel 1e und setzt das Rohgemisch, das die Titelverbindungen enthält, ohne Reigung weiter um.

### Beispiel 1e

### (1S,6S,9S)-4-(4-Benzyloxyphenyl)-9-tert.-butyloxy-1-methyl-bicyclo[4.3.0]non-3-en

8,0 g (26.6 mmol) der nach Beispiel 1c dargestellten Verbindung A versetzt man mit 80 ml einer 50%igen Kaliumhydroxidlösung, 32 ml Benzylchlorid und 1 g Tetrabutyl-ammoniumhydrogensulfat. Man läßt ca. 4 Stunden bei 50°C unter kräftigem Rühren reagieren und setzt das Rohgemisch, das die Titelverbindung enthält, ohne Reigung weiter um.

### Beispiel 1f

### (1S,6S,9S)-4-(4-Benzyloxyphenyl)-9-hydroxy-1-methyl-bicyclo[4.3.0]non-3-en

Die nach Beispiel 1e und 1d erhaltenen Rohprodukte löst man in 300 ml Dioxan, versetzt mit 40 ml einer 4 normalen Salzsäure und erhitzt 16 Stunden auf 80°C. Nach dem Erkalten gießt man in gesättigte Natriumhydrogencarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 1,5 l feinem Kieselgel mit einem Gradientensystem aus n-Hexan und Ethylacetat. Isoliert werden 9,35 g (28,0 mmol, 87% bezogen auf die in Beispiel 1c erhaltenen Produkte A und B) der Titelverbindung als farbloser Schaum.
¹H-NMR (CDCl₃): δ = 0,79 (3H), 1,36-1,62 (3H), 1,65-1,83 (2H), 2,01-2,64 (4H), 2,49 (1H), 3,83 (1H), 5,08 (2H), 6,00 (1H), 6,93 (2H), 7,28-7,52 (7H) ppm.

### Beispiel 1g

### (1S,6S,9S)-4-(4-Benzyloxyphenyl)-9-tert.-butyldimethylsilyloxy-1-methyl-bicylo[4.3.0]non-3-en

Die Lösung von 6,81 g (20,36 mmol) der nach Beispiel 1f dargestellten Verbindung in 60 ml wasserfreiem Dimethylformamid versetzt man unter einer Atmosphäre aus trockenem Argon mit 2,42 g imidazol, 10 ml tert.-Butyldimethylchlorsilan und rührt 16 Stunden bei 23°C. Man gießt in Wasser, extrahiert mehrfach mit Ethylacetat und trocknet die vereinigten organischen Extrakte über Natriumsulfat. Den nach Filtration und Lösungsmittelabzug erhaltenen Rückstand reinigt man durch Chromatographie an ca. 1 l feinem Kieselgel mit einem Gradientensystem aus n-Hexan und Ethylacetat. Isoliert werden 8,71 g (19,4 mmol. 95%) der Titelverbindung als farbloser Schaum.
¹H-NMR (CDCl₃): δ = 0,03 (3H), 0,05 (3H), 0,75 (3H), 0,90 (9H), 1,38-1,78 (4H), 1,87-2,27 (4H), 2,46 (1H), 3,73 (1H), 5,07 (2H), 5,97 (1H), 6,92 (2H), 7,28-7,51 (7H) ppm.

### Beispiel 1h

### (1S,3R,4S,6S,9S)-4-(4-Benzyloxyphenyl)-9-tert.-butyldimethylsilyloxy-3-hydroxy-1-methyl-bicyclo[4.3.0]nonan

Die Lösung von 8,71 g (19,1 mmol) der nach Beispiel 1g dargestellten Verbindung in 225 ml wasserfreiem Tetrahydrofuran versetzt man unter einer Atmosphäre aus trockenem Argon mit 38,5 ml einer 1 molaren Boran-Tetrahydrofuran-Lösung, kühlt nach 2 Stunden auf 0°C, versetzt mit 65 ml einer 5%igen Natronlauge, anschließend mit 30 ml einer 30%igen Wasserstoffperoxidlösung und rührt weitere 30 Minuten. Man verdünnt mit Wasser und Ethylacetat, trennt die organische Phase ab, wäscht mit gesättigter Natriumthiosulfatlösung und trocknet über Natriumsulfat. Den nach Filtration und Lösungsmittelabzug erhaltenen Rückstand reinigt man durch Chromatographie an ca. 1 l feinem Kieselgel mit einem Gradientensystem aus n-Hexan und Ethylacetat. Isoliert werden 8,06 g (17,3 mmol, 90%) der Titelverbindung als farbloser Schaum.
¹H-NMR (CDCl₃): δ = 0,03 (3H), 0,05 (3H), 0,88 (3H), 0,90 (9H), 1,12 (1H), 1,25-1,76 (7H), 1,98 (1H), 2,18 (1H), 2,38 (1H), 3,71 (1H), 3,94 (1H), 5,07 (2H), 6,97 (2H), 7,19 (2H), 7,29-7,51 (5H) ppm.

### Beispiel 1i

### (1S,4S,6S,9S)-4-(4-Benzyloxyphenyl)-9-tert.-butyldimethyisilyloxy-1-methyl-3-oxo-bicyclo[4.3.0]nonan

12 g Chromtrioxid versetzt man bei 0°C mit 50 ml wasserfreiem Dichlormethan, 46 ml Pyridin, rührt 20 Minuten und versetzt mit der Lösung von 8,59 g (18,4 mmol) der nach Beispiel 1 h dargestellten Verbindung in 50 ml wasserfreiem Dichlormethan. Man läßt 5 Stunden bei 0°C unter einer Atmosphäre aus trockenem Argon reagieren, gießt in eine 5%ige Natronlauge, wäscht den Rückstand mit Dichlormethan und die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 1 l feinem Kieselgel mit einem Gradientensystem aus n-Hexan und Ethylacetat. Isoliert werden 7,02 g (15,1 mmol, 82%) der Titelverbindung als farbloser Schaum.
¹H-NMR (CDCl₃): δ = 0,04 (6H), 0,82 (3H), 0,90 (9H), 1,47 (1H), 1,58 (1H), 1,71 (1H), 1,90 (1H), 1,98-2,19 (3H), 2,23 (1H), 2,57 (1H), 3,40 (1H), 3,89 (1H), 5,06 (2H), 6,96 (2H), 7,03 (2H), 7,28-7,49 (5H) ppm.

### Beispiel 1k

### (1S,3S,4S,6S,9S)-4-(4-Benzyloxyphenyl)-9-tert.-butyldimethylsilyloxy-3-hydroxy-1-methyl-3-[4-(5-Chlorpentyloxy)phenyl]-bicyclo[4.3.0]nonan

Die Lösung von 3,91 g 4-Brom-(5-chlorpent-1-oxy)benzol) in 70 ml wasserfreiem Tetrahydrofuran kühlt man unter einer Atmosphäre aus trockenem Argon auf -78°C, versetzt mit 4,52 ml einer 2,5 molaren Lösung von n-Butyllithium in n-Hexan und nach 45 Minuten mit der Lösung von 4,38 g (9,42 mmol) der nach Beispiel 1i dargestellten Verbindung in 70 ml Tetrahydrofuran. Nach 1,5 Stunden gießt man das Reaktionsgemisch in gesättigte Ammoniumchloridlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 1 l feinem Kieselgel mit einem Gradientensystem aus n-Hexan und Ethylacetat. Isoliert werden 4,00 g (6,03 mmol, 64%) der Titelverbindung sowie 1,43 g (33%) Ausgansmaterial jeweils als farbloser Schaum.
¹H-NMR (CDCl₃): δ = -0,03 (3H), 0,00 (3H), 0,86 (9H), 1,13 (3H), 1,45-2,10 (14H), 2,19 (1H), 3,07 (1H), 3,59 (2H), 3,71 (1H), 3,95 (2H), 4,96 (2H), 6,65-6,81 (6H), 7,12 (2H), 7,28-7,43 (5H) ppm.

### Beispiel 1l

### (1S,6S,9S)-4-(4-Benzyloxyphenyl)-3-[4-(5-chlorpentyloxy)phenyl]-9-hydroxy-1-methyl-bicyclo[4.3.0]non-3-en

Die Lösung von 4,0 g (6,03 mmol) der nach Beispiel 1k dargestellten Verbindung in 70 ml Dioxan versetzt man mit 9 ml einer 4N Salzsäure und erhitzt unter Argonatmosphäre 16 Stunden auf 80°C. Nach dem Erkalten versetzt man mit gesättigter Natriumhydrogencarbonatlösung, extrahiert mehrfach mit Dichlormethan und trocknet die vereinigten organischen Extrakte über Natriumsulfat. Den nach Filtration und Lösungsmittelabzug erhaltenen Rückstand reinigt man durch Chromatographie an ca. 400 ml feinem Kieselgel mit einem Gradientensystem aus n-Hexan und Ethylacetat. Isoliert werden 3,01 g (5,67 mmol, 94%) der Titelverbindung als farbloser Schaum.
¹H-NMR (CDCl₃): δ = 0,91 (3H), 1,36-1,68 (5H), 1,68-1,90 (6H), 2,08-2,39 (3H), 2,48 (1H), 2,53 (1H), 3,56 (2H), 3,88 (3H), 4,98 (2H), 6,63 (2H), 6,73 (2H), 6,99 (4H), 7,27-7,46 (5H) ppm.

### Beispiel 1m

### (1S,3S,4R,6S,9S)3-[4-(5-Chlorpentyloxy)phenyl]-9-hydroxy-4-(4-hydroxyphenyl)-1-methyl-bicyclo[4.3.0]nonan

Die Lösung von 1,40 g (2.64 mmol) der nach Beispiel 1l dargestellten Verbindung versetzt man mit 140 mg Palladium auf Kohle (10%ig) und hydriert unter Schütteln bei 1 at Wasserstoff. Man filtriert über Celite, wäscht mit Dichlormethan nach und setzt , den nach Lösungsmittelabzug erhaltenen Rückstand ohne Reingung weiter um. Isoliert werden 1,17 g (2,64 mmol. 100%) der Titelverbindung als farbloser Schaum.
¹H-NMR (CDCl₃+CD₃OD): δ = 0,31 (3H), 1,27-1,82 (11H), 1,93-2,14 (3H), 2,34 (1H), 3,08 (1H). 3,49 (2H), 3,60 (1H), 3,69 (1H), 3,80 (2H), 6,53 (2H), 6,65 (2H), 7,00 (2H), 7,12 (2H) ppm.

### Beispiel 1

### (1S,3S,4R,6S,9S)-9-Hydroxy-4-(4-hydroxyphenyl)-1-methyl-3-[4-(10,10,11,11,11-pentafluor-6-thia-undecyloxy)phenyl]-bicyclo[4.3.0]nonan

Die Lösung von 2,3 g Thioessigsäure-S-(4,4,5,5,5-pentafluor-pentyl)ester in 90 ml Methanol versetzt man unter einer Atmosphäre aus trockenem Argon mit 540 mg Natriumethanolat, läßt 2 Stunden bei 23°C reagieren und erhitzt weitere 2 Stunden auf 40°C. Man läßt auf 23°C abkühlen und tropft die Lösung von 980 mg (2,21 mmol) der nach Beispiel 1m dargestellten Verbindung in 60 ml wasserfreiem Methanol zu, versetzt mit 460 mg Natriumiodid und erhitzt 16 Stunden auf 80°C. Das Reaktionsgemisch gießt man in Wasser, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand reinigt man durch Chromatographie an 200 ml feinem Kieselgel mit einem Gradientensystem aus n-Hexan und Ethylacetat. Isoliert werden 901 mg (1,50 mmol, 68%) der Titelverbindung sowie 300 mg des Ausgangsmaterials.

### Beispiel 2

### (1S,3S,4R,6S,9S)-9-Acetyloxy-4-(4-hydroxyphenyl)-1-methyl-3-[4-(10,10,11,11,11-pentafluor-6-thia-undecyloxy)phenyl]-bicyclo[4.3.0]nonan

### Beispiel 2a

### (1S,6S,9S)-9-Acetyloxy-4-(4-benzyloxyphenyl)-3-[4-(5-chlorpentyloxy)phenyl]-1-methyl-bicyclo[4.3.0]non-3-en

1,50 g (2,82 mmol) der nach Beispiel 1l dargestellten Verbindung verestert man in Analogie zu Beispiel 2d und isoliert nach Aufarbeitung und Reinigung 1,58 g (2,76 mmol. 98%) der Titelverbindung als farblosen Schaum.

### Beispiel 2b

### (1S,3S,4R,6S,9S)-9-Acetyloxy-3-[4-(5-chlorpentyloxy)phenyl]-4-(4-hydroxyphenyl)-1-methyl-bicyclo[4.3.0]nonan

1,58 g (2.76 mmol) der nach Beispiel 2a dargestellten Verbindung setzt man in Analogie zu Beispiel 1m um und isoliert nach Aufarbeitung 1,31g (2,70 mmol, 98%) der Titelverbindung als farblosen Schaum, den man ohne Reinigung weiter umsetzt.
¹H-NMR (CDCl₃): δ = 0,43 (3H), 1,40-1,96 (11H), 2,01 (3H), 2,10-2,26 (3H), 2,31 (1H), 3,14 (1H), 3,53 (2H), 3,72 (1H), 3,84 (2H), 4,63 (1H), 4,68 (1H), 6,58 (2H), 6,69 (2H), 7,02 (2H), 7,18 (2H) ppm.

### Beispiel 2c

### (1S,3S,4R,6S,9S)-9-Acetyloxy-3-[4-(5-chlorpentyloxy)phenyl]-1-methyl-4-[4-(tetrahydropyran-2-yloxy)phenyl]-bicyclo[4.3.0]nonan

Die Lösung von 1,31 g (2,70 mmol) der nach Beispiel 2b dargestellten Verbindung in 35 ml wasserfreiem Dichlormethan versetzt man unter einer Atmosphäre aus trockenem Argon mit 3,3 ml 3,4-Dihydro-(2H)-pyran, 341 mg p-Toluolsulfonsäure-Monohydrat und rührt 4 Stunden bei 23°C. Man gießt in gesättigte Natriumhydrogencarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 300 ml feinem Kieselgel mit einem Gradientensystem aus n-Hexan und Ethylacetat. Isoliert werden 1,13 g (2,51 mmol, 93%) der Titelverbindung als farbloser Schaum.
¹H-NMR (CDCl₃): δ = 0,43 (3H), 1,40-2,05 (17H), 2,02 (3H), 2,10-2,25 (3H), 2,31 (1H), 3,16 (1H), 3,54 (3H), 3,74 (1H), 3,83 (2H), 3,89 (1H), 4,63 (1H), 5,32 (1H), 6,59 (2H), 6,91 (2H), 7,03 (2H), 7,22 (2H) ppm.

### Beispiel 2d

### (1S,3S,4R,6S,9S)-9-Acetyloxy-1-methyl-3-[4-(10,10,11,11,11-pentafluor-6-thiaundecyloxy)phenyl]-4-[4-(tetrahydropyran-2-yloxy)phenyl]-bicyclo[4.3.0]nonan

Die Lösung von 1,43 g (2,51 mmol) der in Beispiel 2c dargestellten Verbindung setzt man in Analogie zu Beispiel 1 um und isoliert nach Aufarbeitung ein Gemisch bestehend aus der Titelverbindung und (1S,3S,4R,6S,9S)-9-Hydroxy-4-[4-(tetrahydropyran-2-yloxy)phenyl]-1-methyl-3-[4-(10,10,11,11,11-pentafluor-6-thia-undecyloxy)phenyl]-bicyclo[4.3.0]nonan, das man in 25 ml wasserfreiem Pyridin löst, mit 3 ml Acetanhydrid, einer Spatelspitze 4-Dimethylaminopyridin versetzt und 2 Stunden bei 23°C rührt. Man gießt in gesättigte Natriumhydrogencarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 400 ml feinem Kieselgel mit einem Gradientensystem aus n-Hexan und Ethylacetat. Isoliert werden 1,57 g (2,16 mmol, 86%) der Titelverbindung als farbloser Schaum.

### Beispiel 2

### (1S,3S,4R,6S,9S)-9-Acetyloxy-4-(4-hydroxyphenyl)-1-methyl-3-[4-(10,10,11,11,11-pentafluor-6-thia-undecyloxy)phenyl]-bicydo[4.3.0]nonan

Die Lösung von 1,57 g (2,16 mmol) der nach Beispiel 2d dargestellten Verbindung in 50 ml wasserfreiem Ethanol versetzt man unter einer Atmosphäre aus trockenem Argon mit 400 mg p-Toluolsulfonsäure-Monohydrat und rührt 1 Stunde bei 23°C. Man gießt in gesättigte Natriumhydrogencarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 300 ml feinem Kieselgel mit einem Gradientensystem aus n-Hexan und Ethylacetat. Isoliert werden 1,12 g (1,74 mmol, 81%) der Titelverbindung als farbloser Feststoff. Kristallisation aus Ethylacetat liefert farblose Kristalle.
¹H-NMR (CDCl₃): δ = 0,43 (3H), 1,40-1,80 (12H), 1,80-1,95 (3H), 2,01 (3H), 2,04-2,37 (6H), 2,51 (2H), 2,59 (2H), 3,14 (1H), 3,72 (1H), 3,83 (2H), 4,64 (1H), 6,58 (2H), 6,69 (2H), 7,02 (2H), 7,18 (2H) ppm.

### Beispiel 3

### (1S,3S,4R,6S,9S)-9-Hydroxy-4-(4-hydroxyphenyl)-1-methyl-3-[4-(5-(4,4,5,5,5-pentafluor-pentylsulfinyl)pentyloxy)phenyl]-bicyclo[4.3.0]nonan

Die Lösung von 200 mg (0,330 mmol) der nach Beispiel 1 dargestellten Verbindung in 8 ml Dichlormethan versetzt man mit 0,88 ml einer 0,5 molaren Natriumhydrogencarbonatlösung, 116 mg 55%iger meta-Chlorperbenzoesäure und rührt 0,5 Stunden bei 23°C. Man gießt in gesättigte Natriumhydrogencarbonatlösung, extrahiert mehrfach mit Dichlormethan, wäscht die vereinigten organischen Extrakte mit gesättigter Natriumchloridtösung und trocknet über Natriumsulfat. Den nach Filtration und Lösungsmittelabzug erhaltenen Rückstand reinigt man durch Chromatographie an ca. 100 ml feinem Kieselgel mit einem Gradientensystem aus n-Hexan und Ethylacetat. Isoliert werden 122 mg (0,20 mmol, 60%) der Titelverbindung als farbloser Schaum.
¹H-NMR (CDCl₃): δ = 0,41 (3H), 1,30-1,88 (12H), 2,01-2,32 (7H), 2,39 (1H), 2,55-2,82 (4H), 3,13 (1H), 3,61-3,78 (2H), 3,85 (2H), 5,74+5,83 (1H), 6,56 (2H), 6,68 (2H), 7,01 (2H), 7,16 (2H) ppm.

### Beispiel 4

### (1S,3S,4R,6S,9S)-9-Hydroxy-4-(4-hydroxyphenyl)-1-methyl-3-[4-(5-(4,4,5,5,5-pentafluor-pentylsulfonyl)pentyloxy)phenyl]-bicyclo[4.3.0]nonan

174 mg (0,29 mmol) der nach Beispiel 1 dargestellten Verbindung setzt man in Analogie zu Beispiel 3 unter Verwendung der doppelten Menge an meta-Chlorperbenzoesäure und 1,5 Stunden Reaktionszeit um und isoliert nach Aufarbeitung und Reinigung 136 mg (0,21 mmol, 74%) der Titelverbindung als farblosen Schaum.
¹H-NMR (CDCl₃): δ = 0,38 (3H), 1,23-1,97 (12H), 2,01-2,45 (8H), 2,90-3,09 (4H), 3,13 (1H), 3,61-3,79 (2H), 3,85 (2H), 4,79 (1H), 6,57 (2H), 6,69 (2H), 7,03 (2H), 7,19 (2H) ppm.

### Beispiel 5

### (1S,3S,4R,6S,9S)-9-Acetyloxy-4-(4-hydroxyphenyl)-1-methyl-3-[4-(5-(4,4,5,5,5-pentafluor-pentylsulfinyl)pentyloxy)phenyl]-bicyclo[4.3.0]nonan

250 mg (0,389 mmol) der nach Beispiel 2 dargestellten Verbindung setzt man in Analogie zu. Beispiel 3 um und isoliert nach Aufarbeitung und Reinigung 149 mg (0,226 mmol, 58%) der Titelverbindung als farblosen Schaum.
¹H-NMR (CDCl₃): δ = 0,47 (3H), 1,41-1,84 (11H), 1,91 (1H), 2,01 (3H), 2,08-2,33 (7H), 2,55-2,81 (4H), 3,14 (1H), 3,70 (1H), 3,88 (2H), 4,64 (1H), 5,62+5,72 (1H), 6,57 (2H), 6,68 (2H), 7,01 (2H), 7,13 (2H) ppm.

### Beispiel 6

### (1S,6S,9S)-9-Hydroxy-4-(4-hydroxyphenyl)-1-methyl-3-[4-(10,10,11,11,11-pentafluor-6-thia-undecyloxy)phenyl]-bicyclo[4.3.0]non-3-en

### Beispiel 6a

### (1S,3S,4S,6S,9S)-9-tert.-Butyldimethylsilyloxy-3-[4-(5-chlorpentyloxy)phenyl]-3-hydroxy-4-(4-hydroxyphenyl)-1-methyl-bicyclo[4.3.0]nonan

9,80 g (14,8 mmol) der nach Beispiel 1k dargestellten Verbindung setzt man in Analogie zu Beispiel 1m um und isoliert nach Aufarbeitung und Reinigung 6,23 g (10,9 mmol, 73%) der Titelverbindung als farblosen Schaum.
¹H-NMR (CDCl₃): δ = -0,03 (3H), 0,01 (3H), 0,87 (9H), 1,17 (3H), 1,44-1,73 (9H), 1,74-2,09 (6H), 2,17 (1H), 3,03 (1H), 3,58 (2H), 3,71 (1H), 3,94 (2H), 4,58 (1H), 6,57 (2H), 6,71 (2H), 6,77 (2H), 7,12 (2H) ppm.

### Beispiel 6b

### (1S,6S,9S)-3-[4-(5-Chlorpentyloxy)phenyl]-9-hydroxy-4-(4-hydroxyphenyl)-1-methyl-bicyclo[4.3.0]non-3-en

5,60 g (9,77 mmol) der nach Beispiel 6a dargestellten Verbindung setzt man in Analogie zu Beispiel 1l um und isoliert nach Aufarbeitung und Reinigung 4,11 g (9,32 mmol, 95%) der Titelverbindung als farblosen Schaum.
¹H-NMR (CDCl₃): δ = 0,91 (3H), 1,35-1,68 (5H), 1,68-1,90 (6H), 2,08-2,38 (3H), 2,41-2,58 (2H), 3,54 (2H), 3,89 (3H), 4,77 (1H), 6,57 (2H), 6,62 (2H), 6;82 (2H), 6,87 (2H) ppm.

### Beispiel 6

### (1S,6S,9S)-9-Hydroxy-4-(4-hydroxyphenyl)-1-methyl-3-[4-(10,10,11,11,11-pentafluor-6-thia-undecyloxy)phenyl]-bicyclo[4.3.0]non-3-en

4,11 g (9,32 mmol) der nach Beispiel 6b dargestellten Verbindung setzt man in Analogie zu Beispiel 1 um und isoliert nach Aufarbeitung und Reinigung 4,32 g (7,22 mmol, 77%) der Titelverbindung als farblosen Schaum.
¹H-NMR (CDCl₃): δ = 0,91 (3H), 1,36-1,97 (13H), 2.07-2,38 (5H), 2,41-2,64 (6H), 3,87 (3H), 4,68 (1H); 6,57 (2H), 6,63 (2H), 6,83 (2H), 6,87 (2H) ppm.

### Beispiel 7

### (1S,6S,9S)-9-Acetyloxy-4-(4-hydroxyphenyl)-1-methyl-3-[4-(10,10,11,11,11-pentafluor-6-thia-undecyloxy)phenyl]-bicyclo[4.3.0]non-3-en

### Beispiel 7a

### (1S,6S,9S)-4-(4-tert.-Butyldimethylsilyloxyphenyl)-9-hydroxy-1-methyl-3-[4-(10,10,11,11,11-pentafluor-6-thia-undecyloxy)phenyl]-bicyclo[4.3.0]non-3-en

Die Lösung von 2,08 g (3,47 mmol) der nach Beispiel 6 dargestellten Verbindung in 50 ml wasserfreiem Tetrahydrofuran kühlt man unter einer Atmosphäre aus trockenem Argon auf 0°C, versetzt mit 157 mg einer 55%igen Natriumhydrid-Dispersion, 1,5 ml einer 3 molaren Lösung von tert.-Butyldimethylchlorsilan in n-Hexan und rührt 1,5 Stunden bei 23°C. Man gießt in Wasser, extrahiert mit Ethylacetat, wäscht die organische Phase mit gesättigter Natriumchloridlösung und trocknet über Natriumsulfat. Den nach Filtration und Lösungsmittelabzug erhaltenen Rückstand setzt man ohne Reinigung weiter um. Isoliert werden 2,7 g (max. 3,47 mmol) der Titelverbindung.
¹H-NMR (CDCl₃): δ = 0,11 (6H), 0,89 (3H), 0,93 (9H), 1,37-1,93 (13H), 2,03-2,36 (5H), 2,4-2,63 (6H), 3,85 (3H), 6,56 (2H), 6,59 (2H), 6,80 (2H), 6,84 (2H) ppm.

### Beispiel 7b

### (1S,6S,9S)-9-Acetyloxy-4-(4-tert.-butyldimethylsilytoxyphenyl)-1-methy-3-[4-(10,10,11,11,11-pentafluor-6-thia-undecyloxy)phenyl]-bicyclo[4.3.0]non-3-en

2,7 g (max. 3,47 mmol) der nach Beispiel 7a dargestellten Verbindung verestert man in Analogie zu Beispiel 2d und isoliert nach Aufarbeitung 3,0 g der Titelverbindung, die man ohne Reinigung weiter umsetzt.

### Beispiel 7

### (1S,6S,9S)-9-Acetyloxy-4-(4-hydroxyphenyl)-1-methyl-3-[4-(10,10,11,11,11-pentafluor-6-thia-undecyloxy)phenyl]-bicyclo[4.3.0]non-3-en

Die Lösung von 3,0 g (max. 3,47 mmol) der nach Beispiel 7b dargestellten Verbindung in 200 ml wasserfreiem Tetrahydrofuran versetzt man unter einer Atmosphäre aus trockenem Argon mit 3,5 ml einer 1 molaren Lösung von Tetrabutylammoniumfluorid in Tetrahydrofuran und rührt 3 Stunden bei 23°C. Man gießt in gesättigte Ammoniumchloridlösung. extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 600 ml feinem Kieselgel mit einem Gradientensystem aus n-Hexan und Ethylacetat. Isoliert werden 1,87 g (2,92 mmol, 85% bezogen auf Startmaterial in Beispiel 7a) der Titelverbindung als farbloser Schaum.
¹H-NMR (CDCl₃): δ = 0,93 (3H), 1.39-1,97 (12H), 2,02-2,64 (11H); 2,07 (3H), 3,87 (2H), 4,62 (1H), 4,82 (1H), 6,55 (2H), 6,62 (2H), 6,83 (4H) ppm.

### Beispiel 8

### (1S,6S,9S)-9-Acetyloxy-4-(4-hydroxyphenyl)-1-methyl-3-[4-(5-(4,4,5,5,5-pentafuor-pentylsufonyl)pentyloxy)phenyl]-bicyclo[4.3.0]non-3-en

500 mg (0,78 mmol) der nach Beispiel 7 dargestellten Verbindung setzt man in Analogie zu Beispiel 4 um und isoliert nach Aufarbeitung und Reinigung 308 mg (0,46 mmol, 59%) der Titelverbindung als farblosen Schaum.
¹H-NMR (CDCl₃): δ = 0,95 (3H), 1,41-1,97 (10H), 2,07 (3H), 2,11-2,43 (8H), 2,51 (1H), 2,92-3,09 (4H), 3,91 (2H), 4,82 (1H), 4,91 (1H), 6,56 (2H), 6,61 (2H), 6,81 (2H), 6,86 (2H) ppm.

### Beispiel 9

### (1S,6S,9S)-9-Acetyloxy-4-(4-hydroxyphenyl)-1-methyl-3-[4-(5-(4,4,5,5,5-pentafluor-pentylsulfinyl)pentyloxy)phenyl]-bicyclo[4.3.0]non-3-en

310 mg (0,48 mmol) der nach Beispiel 7 dargestellten Verbindung setzt man in Analogie zu Beispiel 3 um und isoliert nach Aufarbeitung und Reinigung 255 mg (0,39 mmol, 80%) der Titelverbindung als farblosen Schaum.
¹H-NMR (CDCl₃): δ = 0.95 (3H), 1,40-1,96 (10H), 2,02 (3H), 2,07-2,84 (13H), 3,87-4,02 (2H), 4,81 (1H), 6.55 (2H), 6,61 (2H), 6,77 (2H), 6,83 (2H), 6,93 (1H) ppm.

### Beispiel 10

### (1S,6S,9S)-9-Hydroxy-4-(4-hydroxyphenyl)-1-methyl-3-[4-(5-(4,4,5,5,5-pentafluor-pentylsulfonyl)pentyloxy)phenyl]-bicyclo[4.3.0]non-3-en

500 mg (0,835 mmol) der nach Beispiel 6 dargestellten Verbindung setzt man in Analogie zu Beispiel 4 um und isoliert nach Aufarbeitung und Reinigung 290 mg (0,46 mmol, 55%) der Titelverbindung als farblosen Schaum.
¹H-NMR (CDCl₃): δ = 0,91 (3H), 1,37-1,98 (11H), 2,09-2,53 (9H), 2,92-3,10 (4H), 3,87 (1H), 3,91 (2H), 4,95 (1H), 6,56 (2H), 6,61 (2H), 6,81 (2H), 6,87 (2H) ppm.

### Beispiel 11

### (1S,3S,4R,6S,9S)-3,4-Epoxy-9-hydroxy-4-(4-hydroxyphenyl)-1-methyl-3-[4-(5-(4,4,5,5,5-pentafluor-pentylsulfonyl)pentyloxy)phenyl]-bicyclo[4.3.0]nonan

151 mg (0,24 mmol) der nach Beispiel 10 dargestellten Verbindung setzt man in Analogie zu Beispiel 3 um und isoliert nach Aufarbeitung und Reinigung 92 mg (0,14 mmol, 59%) der Titelverbindung als farblosen Schaum.
¹H-NMR (CDCl₃): δ = 1,12 (3H), 1,40-2,43 (20H), 2,91-3,11 (4H), 3,79 (1H), 3,88 (2H), 5,13 (1H), 6,53 (2H), 6,59 (2H), 6,96 (2H), 7,01 (2H) ppm.

### Beispiel 12

### (1S,6S,9S)-9-Hydroxy-4-(4-hydroxyphenyl)-1-methyl-3-[4-(5-(4,4,5,5,5-pentafluor-pentylsulfinyl)pentyloxy)phenyl]-bicyclo[4.3.0]non-3-en

500 mg (0,835 mmol) der nach Beispiel 6 dargestellten Verbindung setzt man in Analogie zu Beispiel 3 um und isoliert nach Aufarbeitung und Reinigung 357 mg (0,58 mmol, 70%) der Titelverbindung als farblosen Schaum.
¹H-NMR (CDCl₃ + CD₃OD): δ = 0,88 (3H), 1,33-1,85 (9H), 2,01-2,31 (7H), 2,37-2,81 (8H), 3,80 (1H), 3,88 (2H), 6,52 (2H), 6,58 (2H), 6,74 (2H), 6,83 (2H) ppm.

### Beispiel 13

### (1S,3S,'4R,6S,9S)9-Acetoxy-3,4-epoxy-4-(4-hydroxyphenyl)-1-methyl-3-[4-(5-(4,4,5,5,5-pentafluor-pentylsulfonyl)pentyloxy)phenyl]-bicyclo[4.3.0]nonan

110 mg (0,16 mmol) der nach Beispiel 8 dargestellten Verbindung setzt man in Analogie zu Beispiel 3 um und isoliert nach Aufarbeitung und Reinigung 81 mg (0,12 mmol, 50%) der Titelverbindung als farblosen Schaum.
¹H-NMR (CDCl₃): δ = 1,40-2,38 (19H), 2,06 (3H), 2,90-3,08 (4H), 3,89 (2H), 4,71 (1H), 5,08 (1H), 6,53 (2H), 6,59 (2H), 6,97 (2H), 6,99 (2H) ppm.

### Beispiel 14

### (1S,6S,9S)-9-Hydroxy-4-(4-hydroxyphenyl)-1-methyl-3-[4-(2-chlorethyloxy)phenyl]-bicyclo[4.3.0]non-3-en

### Beispiel 14a

### (1S,6S,9S)-9-Hydroxy-4-(4-hydroxyphenyl)-1-methyl-bicyclo[4.3.0]non-3-en

12,8 g (42,7 mmol) der nach Beispiel 1a dargestellten Verbindung A setzt man in Analogie zu Beispiel 1f um und isoliert nach Aufarbeitung und Reinigung 8,5 g (34,8 mmol, 81%) der Titelverbindung als farblosen Schaum.

### Beispiel 14b

### (1S,6S,9S)-9-(tert.-Butyldimethylsilyloxy)-4-(4-tert.-butyldimethylsilyloxyphenyl)-1-methyl-bicyclo[4.3.0]non-3-en

8,5 g (34,8 mmol) der nach Beispiel 14a dargestellten Verbindung setzt man in Analogie zu Beispiel 1g um und isoliert nach Aufarbeitung und Reinigung 15,0 g (31,7 mmol, 91%) der Titelverbindung als farblosen Schaum.
¹H-NMR (CDCl₃): δ = 0,04 (6H), 0,19 (6H), 0,73 (3H), 0,90 (9H), 0,98 (9H), 1,38-1,78 (4H), 1,87-2,03 (2H), 2,08-2,25 (2H), 2,44 (1H), 3,73 (1H), 5,98 (1H), 6,78 (2H), 7,28 (2H) ppm.

### Beispiel 14c

### (1S,3R,4S,6S,9S)-9-(tert.-Butyldimethylsilyloxy)-3-hydroxy-4-(4-tert.butyldimethylsilyloxyphenyl)-1-methyl-bicyclo[4.3.0]nonan

15,0 g (31,7 mmol) der nach Beispiel 14b dargestellten Verbindung setzt man in Analogie zu Beispiel 1h um und isoliert nach Aufarbeitung und Reinigung 14,4 g (29,3 mmol, 93%) der Titelverbindung als farblosen Schaum.
¹H-NMR (CDCl₃): δ = 0,03 (6H), 0,19 (6H), 0,87 (3H), 0,89 (9H), 0.99 (9H), 1.12 (1H), 1,22-1,74 (7H), 1,97 (1H), 2,17 (1H), 2,36 (1H), 3,71 (1H), 3,92 (1H), 6,81 (2H), 7,12 (2H) ppm.

### Beispiel 14d

### (1S,4S,6S,9S)-9-(tert.-Butyldimethylsilyloxy)-4-(4-tert.butyldimethylsilyloxyphenyl)-1-methyl-3-oxo-bicyclo[4.3.0]nonan

14,4 g (29,3 mmol) der nach Beispiel 14c dargestellten Verbindung setzt man in Analogie zu Beispiel 1i um und isoliert nach Aufarbeitung und Reinigung 9,49 g (19,4 mmol, 66%) der Titelverbindung als farblosen Schaum.
¹H-NMR (CDCl₃): δ = 0,03 (6H), 0,19 (6H), 0,79 (3H), 0,88 (9H), 0,97 (9H), 1,44 (1H), 1,55 (1H), 1,69 (1H), 1,87 (1H), 1,96-2,17 (3H), 2,21 (1H), 2,53 (1H), 3,38 (1H), 3,87 (1H), 6,79 (2H), 6,96 (2H) ppm.

### Beispiel 14e

### (1S,6S,9S)-9-(tert.-Butyldimethylsilyloxy)-4-(4-tert.-butyldimethylsilyloxyphenyl)-1-methyl-3-(nonafluorbutylsulfonyloxy)-bicyclo[4.3.0]non-3-en

Die Lösung von 5,0 g (10,2 mmol) der nach Beispiel 14d dargestellten Verbindung und 3,71 g Kalium-bis-(trimethylsilyl)amid in 95 ml wasserfreiem Tetrahydrofuran versetzt man bei 0°C mit 2,42 ml Perfluorbutansulfonylfluorid und rührt 1,5 Stunden. Man gießt auf Natriumhydrogencarbonatlösung, extrahiert mehrfach mit Ethylacetat und trocknet die vereinigten organischen Extrakte über Natriumsulfat. Den nach Filtration und Lösungsmittelabzug erhaltenen Rückstand setzt man ohne Reinigung weiter um.

### Beispiel 14f

### (11S,6S,9S)-9-(tert.-Butyldimethylsilyloxy)-4-(4-tert.-butyldimethyisilyloxyphenyl)-1-methyl-3-[4-(2-chlorethyloxy)phenyl]-bicyclo[4.3.0]non-3-en

Die Lösung des nach Beispiel 14e dargestellten Rohproduktes in einem Gemisch aus 150 ml Toluol und 66 ml Ethanol versetzt man unter einer Atmosphäre aus Argon mit 955 mg Lithiumchlorid, 15 ml einer 2 M Natriumcarbonatlösung, 1,0 g [4-(2-chlorethyloxy)phenyl]-boronsäure, 1,0g Tetrakis-triphenylphosphin-palladium (0) und erwärmt 1,5 Stunden auf 95°C. Man verdünnt mit Wasser, extrahiert mehrfach mit Ethylacetat und trocknet die vereinigten organischen Extrakte über Natriumsulfat. Den nach Filtration und Lösungsmittelabzug erhaltenen Rückstand reinigt man durch Chromatographie an ca. 1,5 l feinem Kieselgel mit einem Gradientensystem aus n-Hexan und Ethylacetat. Isoliert werden 2,15 g (3,43 mmol, 34% bezogen auf Startmaterial in Beispiel 14e) der Titelverbindung als farbloser Schaum sowie 1,82 g der in Beispiel 14d erwähnten Titelverbindung.
¹H-NMR (CDCl₃): δ = 0,02 (6H), 0,12 (6H), 0,86 (3H), 0,89 (9H), 0,92 (9H), 1,32-1,81 (5H), 1,89-2,54 (4H), 3,69-3,85 (3H), 4,13 (2H), 6,57 (2H), 6,63 (2H), 6,80 (2H), 6,88 (2H) ppm.

### Beispiel 14g

### (1S,4S,6S,9S)- 4-(4-Hydroxyphenyl)-1-methyl-3-oxo-bicyclo[4.3.0]nonan-9-0

940 mg (1,92 mmol) der nach Beispiel 14d dargestellten bzw. aus Beispiel 14f zurückgewonnen Verbindung setzt man in Analogie zu Beispiel 14 (Variante l) um und isoliert nach Aufarbeitung und Reinigung 415 mg (1,59 mmol, 83%) der Titelverbindung als farblosen Schaum.

### Beispiel 14h

### (1S,4S,6S,9S)-1-Methyl-3-oxo-9-(tetrahydropyran-2-yloxy)-4-[4-(tetrahydropyran-2-yloxy)phenyl]-bicyclo[4.3.0]nonan

Die Lösung von 415 mg (1,59 mmol) der nach Beispiel 14g dargestellten Verbindung in 10 ml wasserfreiem Dichlormethan versetzt man unter einer Atmosphäre aus trockenem Argon mit 0,73 ml 3,4-Dihydro-2H-pyran, 80 mg p-Toluotsulfonsäure-Pyridiniumsalz und rührt 16 Stunden bei 23°C. Man versetzt mit gesättigter Natriumhydrogencarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 300 ml feinem Kieselgel mit einem Gradientensystem aus n-Hexan und Ethylacetat. Isoliert werden 548 mg (1,27 mmol, 81%) der Titelverbindung als farbloser Schaum.
¹H-NMR (CDCl₃): δ = 0,87+0,89 (3H), 1,39-2,30 (19H), 2,38 (1H), 2,62+2,73 (1H), 3,36-3,65 (3H), 3,78-4,01 (3H), 4,61+4,68 (1H), 5,41 (1H), 7,03 (4H) ppm.

### Beispiel 14i

### (1S,6S,9S)-1-methyl-3-(nonafluorbutylsulfonyloxy)-9-(tetrahydropyran-2-yloxy)-4-[4-(tetrahydropyran-2-yloxy)phenyl]-bicyclo[4.3.0]non-3-en

543 mg (1,27 mmol) der nach Beispiel 14h dargestellten Verbindung setzt man in Analogie zu Beispiel 14e um und setzt das nach Aufarbeitung erhaltene Rohprodukt ohne Reinigung weiter um.

### Beispiel 14k

### (1S,6S,9S)-9-(tetrahydropyran-2-yloxy)-4-[4-(tetrahydropyran-2-yloxy)phenyl]-1-methyl-3-[4-(2-chlorethyloxy)phenyl]-bicyclo[4.3.0]non-3-en

Das nach Beispiel 14i dargestellte Rohprodukt setzt man in Analogie zu Beispiel 14f um und isoliert nach Aufarbeitung und Reinigung 181 mg (0.32 mmol, 25% bezogen auf Ausgangsmaterial in Beispiel 14i)der Titelverbindung als farblosen Schaum sowie 350 mg der in Beispiel 14h genannten Titelverbindung.

### Beispiel 14

### (1S,6S,9S)-9-Hydroxy-4-(4-hydroxyphenyl)-1-methyl-3-[4-(2-chlorethyloxy)phenyl]-bicyclo[4.3.0]non-3-en

### Variante I

Die Lösung von 2,14 g (3,42 mmol) der nach Beispiel 14f dargestellten Verbindung in 100 ml Aceton versetzt man mit 5 ml einer 4N Salzsäure und rührt unter Argonatmosphäre 1 Stunde bei 23°C. Man versetzt mit gesättigter Natriumhydrogencarbonatlösung, extrahiert mehrfach mit Dichlormethan und trocknet die vereinigten organischen Extrakte über Natriumsulfat. Den nach Filtration und Lösungsmittelabzug erhaltenen Rückstand reinigt man durch Chromatographie an ca. 300 ml feinem Kieselgel mit einem Gradientensystem aus n-Hexan und Ethylacetat. isoliert werden 642 mg (1,16 mmol, 34%) der Titelverbindung als farbloser Schaum.

### Variante II

181 mg (0,32 mmol) der nach Beispiel 14k dargestellten Verbindung setzt man in Analogie zu Beispiel 14 (Variante I) um und isoliert nach Aufarbeitung und Reinigung die Titelverbindung als farblosen Schaum.
¹H-NMR (CDCl₃): δ = 0,92 (3H), 1,34-1,90 (6H), 2,09-2,58 (5H), 3,77 (2H), 3,88 (1H), 4,13 (2H), 6,58 (2H), 6,66 (2H), 6,83 (2H), 6,89 (2H) ppm.

### Beispiel 15

### (1S,6S,9S)-9-Hydroxy-4-(4-hydroxyphenyl)-1-methyl-3-[4-(2-(dimethylamino)ethyloxy)phenyl]-bicyclo[4.3.0]non-3-en

10 ml Ethanol sättigt man mit Dimethylamin, versetzt mit der Lösung von 637 mg (1,60 mmol) der nach Beispiel 14 dargestellten Verbindung in 5 ml Ethanol und rührt 2,5 Tage bei 80°C unter einer Atmosphäre aus trockenem Argon. Man versetzt mit Wasser, einer 5%igen Natronlauge, extrahiert mehrfach mit Trichlormethan und trocknet die vereinigten organischen Extrakte über Natriumsulfat. Nach Filtration und Lösungsmittelabzug isoliert man 584 mg (1,43 mmol, 90%) der Titelverbindung als farblosen Schaum.
¹H-NMR (CDCl₃): δ = 0,91 (3H), 1,23-1,64 (3H), 1,70-1,89 (2H), 2,00 (1H), 2,09-2,57 (5H), 2,49 (6H), 2,79 (2H), 3,88 (1H), 4,01 (2H), 6,55 (2H), 6,58 (2H), 6,79 (2H), 6,86 (2H) ppm.

### Beispiel 16

### (1S,3S,4R,6S,9S)-9-Hydroxy-4-(4-hydroxyphenyl)-1-methyl-3-[4-(2-(dimethylamino)ethyloxy)phenyl]-bicyclo[4.3.0]nonan

Die Lösung von 100 mg (0,245 mmol) der nach Beispiel 15 dargestellten Verbindung in 4 ml Ethanol versetzt man portionsweise mit insgesamt 70 mg Palladium auf Kohle (10% ig) und hydriert 10 Tage bei 200 bar. Nach Filtration reinigt man das Rohprodukt chromatographisch. Isoliert werden 67 mg (165 µmol, 67%) der Titelverbindung als farblosen Schaum.
¹H-NMR (CDCl₃/CD₃OD): δ = 0,32 (3H), 1,28-1,69 (4H), 1,78 (1H), 1,98-2,17 (3H), 2,32 (1H), 2,81 (6H), 3,09 (1H), 3,32 (2H), 3,57-3,73 (2H), 4,10 (2H), 6,49 (2H), 6,65 (2H), 7,03 (2H), 7,11 (2H) ppm.

### Beispiel 17

### (1S,3S,4R,6S,9S)-9-Acetyloxy-4-(4-hydroxyphenyl)-1-methyl-3-[4-(10,10,11,11,11-pentafluorpentylsulfonyl)pentyloxy)phenyl]-bicyclo[4.3.0]nonan

250 mg (0,389 mmol) der nach Beispiel 2 dargestellten Verbindung setzt man in Analogie zu Beispiel 3 um und isoliert nach Aufarbeitung und Reinigung 144 mg (0,213 mmol, 55%) der Titelverbindung als farblosen Feststoff.
¹H-NMR (CDCl₃): δ = 0,44 (3H), 1,40-1,81 (8H), 1,82-1,97 (3H), 2,00 (3H), 2,08-2,38 (8H), 2,90-3,09 (4H), 3,14 (1H), 3,70 (1H), 3,85 (2H), 4,63 (1H), 4,73 (1H), 6,57 (2H), 6,69 (2H), 7,02 (2H), 7,18 (2H) ppm.

### Beispiel 18

### (1S,3S,4R,6S,9S)-9-Hydroxy-4-(4-hydroxyphenyt)-1-methyl-3-[4-(2-chiorethyloxy)phenyl]-bicyclo[4.3.0]nonan

50 mg (0,125 mmol) der nach Beispiel 14 dargestellten Verbindung setzt man in Analogie zu Beispiel 1m um und isoliert nach Aufarbeitung und Reinigung 41 mg (0,102 mmol, 82%) der Titelverbindung als farblosen Schaum.
¹H-NMR (CDCl₃/CD₃OD): δ = 0,31 (3H), 1,29-1,68 (4H), 1,76 (1H), 1,95-2,17 (3H), 2,36 (1H), 3,09 (1H), 3,56-3,75 (2H), 3,70 (2H), 4,07 (2H), 6,56 (2H), 6,65 (2H), 7,02 (2H), 7,12 (2H) ppm.

### Beispiel 19

### (1S,3S,4R,6S,9S)-3-[4-(5-Chlorpentyloxy)phenyl]-3,4-epoxy-9-hydroxy-4-(4-hydroxyphenyl)-1-methyl-bicyclo[4.3.0]nonan

1,00 g (2,27 mmol) der nach Beispiel 6b dargestellten Verbindung setzt man in Analogie zu Beispiel 3 um und isoliert nach Aufarbeitung und Reinigung 597 mg (1,31 mmol, 58%) der Titelverbindung als farblosen Schaum.
¹H-NMR (CDl₃/CD₃OD): δ = 1,07 (3H), 1,32-2,22 (15H), 2,33 (1H), 3,50 (2H), 3,70 (1H), 3,79 (2H), 6,51 (2H), 6,56 (2H), 6,92 (2H), 6,98 (2H) ppm.

### Beispiel 20

### (1S,3S,4R,6S,9S)-3-[4-(10,10,1 1,1 1-pentafluor-6-thia-undecyloxy)phenyl]-3,4-epoxy-9-hydroxy-4-(4-hydroxyphenyl)-1-methyl-bicyclo[4.3.0]nonan (A) und (1S,3S,6S,9S)-3-[4-(10,10,11,11,11-pentafluor-6-thia-undecyloxy)phenyl]-3,9-dihydroxy-4-(4-hydroxyphenyl)-1-methyl-bicyclo[4.3.0]non-4-en (B)

592 mg (1,30 mmol) der nach Beispiel 19 dargestellten Verbindung setzt man in Analogie zu Beispiel 1 um und isoliert nach Aufarbeitung und Reinigung 259 mg (0,42 mmol, 32%) der Titelverbindung A sowie 140 mg (0,23 mmol, 18%) der Titelverbindung B jeweils als farblosen Schaum.
¹H-NMR (CDCl₃) von A: δ = 1,12 (3H), 1,34-1,78 (10H), 1,78-2,27 (9H), 2,38 (1H), 2,51 (2H), 2,59 (2H), 3,80 (1H), 3,83 (2H), 4,68 (1H), 6,55 (2H), 6,61 (2H), 7,00 (4H) ppm.
¹H-NMR (CDCl₃) von B: δ = 0,70 (3H), 1,34 (1H), 1,45-1,94 (11H), 2,02-2,28 (5H), 2,39-2,64 (6H), 3,87 (1H), 3,90 (2H), 4,81 (1H), 6.23 (1H), 6,61 (2H), 6,71 (2H), 7,29 (4H) ppm.

### Beispiel 21

### (1S,3S,4R,6S,9S)-3-[4-(5-(4,4,5,5,5-pentafluor-pentylsulfinyl)pentyloxy)phenyl-3,4-epoxy-9-hydroxy-4-(4-hydroxyphenyl)-1-methyl-bicyclo[4.3.0]nonan

149 mg (0,24 mmol) der nach Beispiel 20 dargestellten Verbindung setzt man in Analogie zu Beispiel 3 um und isoliert nach Aufarbeitung und Reinigung 64 mg (0,10 mmol, 42%) der Titelverbindung als farblosen Schaum.
¹H-NMR (CDCl₃): δ = 1,11 (3H), 1,33-1,76 (10H), 1,80-2,33 (9H), 2,41 (1H), 2,53-2,88 (4H), 3,80 (1H), 3,89-4,04 (2H), 6,52 (2H), 6,59 (2H), 6,90 (2H), 7,00 (2H), 7,170+7,26 (1H) ppm.

### Beispiel 22

### (1S,3S,4R,6S,9S)-9-Hydroxy-4-(4-hydroxyphenyl)-1-methyl-3-[4-(2-(pentamethylenamino)ethyloxy)phenyl]-bicyclo[4.3.0]nonan

Die Lösung von 31 mg (77µmol) der nach Beispiel 18 dargestellten Verbindung setzt man in Analogie zu Beispiel 15 unter Verwendung von Piperidin um und isoliert nach Aufarbeitung und Reinigung 24 mg (53 µmol, 69%) der Titelverbindung als blassgelben Schaum.
¹H-NMR (CDCl₃/CD₃OD): δ = 0,33 (3H), 1,28-1,70 (11H), 1,77 (1H), 1,98-2,18 (3H), 2,35 (1H), 2,55 (4H), 2,74 (2H), 3,09 (1H), 3,58-3,74 (2H), 3,96 (2H), 6,52 (2H), 6,68 (2H), 7,01 (2H), 7,12 (2H) ppm.

## Patentansprüche

1. 3,4-Diphenyl-bicyclo[4.3.0]nonyl-Derivate der allgemeinen Formel l 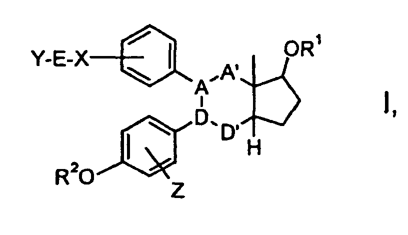 worin
R¹ für gegebenenfalls substituiertes C₁-C₂₀-Alkanoyl, gegebenenfalls substituiertes C₁-C₂₀-Alkyl, gegebenenfalls substituiertes C₇-C₂₀-Aralkyl, gegebenenfalls substituiertes C₇-C₁₅-Aroyl, eine Gruppe PG¹ oder ein Wasserstoffatom,
R² für gegebenenfalls substituiertes C₁-C₂₀-Alkanoyl, gegebenenfalls substituiertes C₁-C₂₀-Alkyl, gegebenenfalls substituiertes C₇-C₂₀-Aralkyl, gegebenenfalls substituiertes C₇-C₁₅-Aroyl, eine Gruppe PG² oder ein Wasserstoffatom,
PG¹, PG² gleich oder verschieden sind und für eine Schutzgruppe PG, wobei PG ein Methoxymethyl-, Methoxyethyl-, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Methyl-, tert.-Butyl-, Benzyl-, para-Nitrobenzyl-, para-Methoxybenzyl, Formyl-, Acetyl-, Propionyl-, Butyryl-, Pivaloyl-, Benzoyl-Rest ist,
A'-A-D-D' für eine -CH₂-C(OH)-C=CH-, -CH=C-C(OH)-CH₂-, -CH=C-C=CH-, -CH₂-C=C-CH₂-, -CH₂-CH-CH-CH₂-, -CH₂-C(OH)-CH-CH₂-, -CH₂-CH-C(OH)-CH₂-, -CH₂-C(OH)-C(OH)-CH₂- oder 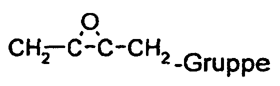 (Hydroxy= α oder β; Epoxy = α oder β),
X für eine Bindung, ein Sauerstoffatom, ein Schwefelatom, SO oder SO₂ ,
E für eine gerad- oder verzweigtkettige Alkylen-, Alkenylen- oder Alkinylengruppe mit bis zu 15 Kohlenstoffatomen,
Y für Halogen (F, Cl, Br, I), für einen Substituenten R⁴, einen gegebenenfalls substituierten Aryl- oder Heteroarylrest. eine NR^{4a}R^{4b}-, SO₂NR^{4a}R^{4b}-, NR^{4a}(CH₂)ₚ-Q-G-, NR⁵(CHR⁶-CHR⁷)-(CH₂)ₜ-Q-G-, SO₂NR^{4a}(CH₂)ₚ-Q-G-, eine O-G-, S-G-, SO-G-, SO₂-G-Gruppe,
R⁴ für ein Wasserstoffatom, gegebenenfalls substituiertes C₁-C₂₀-Alkyl, teilweise oder vollständig fluoriertes C₁-C₂₀-Alkyl, gegebenenfalls substituiertes C₁-C₂₀-Alkanoyl, gegebenenfalls substituiertes Aryl, gegebenenfalls substituiertes Heteroaryl, gegebenenfalls substituiertes C₇-C₂₀-Aralkyl, gegebenenfalls substituiertes C₇-C₁₅-Aroyl,
Q für ein Sauerstoffatom, ein Schwefelatom, SO oder SO₂
G für -(CH₂)ₙ-R³,
n für 0 bis 10,
p für 1 bis 10,
t für 0,1 oder 2
R³ für Wasserstoff, eine gerad- oder verzweigtkettige Alkyl-, Alkenyl- oder Alkinylgruppe mit bis zu 10 Kohlenstoffatomen, eine gerad- oder verzweigtkettige, teilweise oder vollständig fluorierte Alkyl- oder Alkenylgruppe mit bis zu 10 Kohlenstoffatomen, eine gegebenenfalls substituierte C₄-C₈-Cycloalkyl-, eine gegebenenfalls substituierte Aryl-, eine gegebenenfalls substituierte C₇-C₂₀-Aralkylgruppe oder, falls n>0 ist, auch für eine Hydroxygruppe oder ein Halogenatom,
R^{4a}, R^{4b} gleich oder verschieden sind in der Bedeutung von R⁴ oder gemeinsam für eine C₃-C₁₅-Alkylengruppe, die geradkettig oder verzweigt sein kann
R⁵ ein Wasserstoffatom oder eine C₁₋₅-Alkylgruppe,
R⁶ und R⁷ je ein Wasserstoffatom, oder
R⁵ und R⁶ gemeinsam eine Alkylengruppe ―(CH₂)_{d}― mit d = 2, 3, 4 oder 5 und R⁷ ein Wasserstoffatom oder
R⁵ und R⁷ gemeinsam eine Alkylengruppe ―(CH₂)ₑ― mit e = 2, 3 oder 4 und R⁶ ein Wasserstoffatom.
Z für Wasserstoff, Halogen, OH, N₃, NH₂, CO₂H, CO₂-(C₁-C₂₀)-Alkyl, C₁-C₂₀-Alkoxy, -NO₂, -CN, oder C₁-C₂₀-Acyloxy.
stehen.

2. Verbindungen der allgemeinen Formel I nach Anspruch 1, worin R¹ und / oder R² für ein Wasserstoffatom stehen.

3. Verbindungen der allgemeinen Formel I nach Anspruch 1, worin A'-A-D-D' für eine -CH₂-CH-CH-CH₂--Gruppe steht.

4. Verbindungen der allgemeinen Formel I nach Anspruch 1, worin A'-A-D-D' für eine -CH₂-C=C-CH₂--Gruppe steht.

5. Verbindungen der allgemeinen Formel I nach Anspruch 1, worin A'-A-D-D' für eine 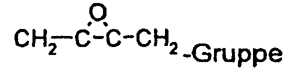 (Epoxy = α) steht.

6. Verbindungen der allgemeinen Formel I nach Anspruch 1, worin A'-A-D-D' für eine -CH₂-C(OH)-C=CH―Gruppe steht.

7. Verbindungen der allgemeinen Formel I, worin die Seitenkette -X-E-Y aus der Gruppe der folgenden Seitenketten ausgewählt ist
-O-(CH₂)₅S(CH₂)₃C₂F₅
-O-(CH₂)₅SO(CH₂)₃C₂F₅
-O-(CH₂)₅SO₂(CH₂)₃C₂F₅
-O-(CH₂)₄S(CH₂)₃C₂F₅
-O-(CH₂)₄SO(CH₂)₃C₂F₅
-O-(CH₂)₄SO₂(CH₂)₃C₂F₅
-O-(CH₂)₅-Cl
-O-(CH₂)₄-Cl
-O-(CH₂)₃-Cl
-O-(CH₂)₂-Cl
-O-(CH₂)₂-N(CH₃)₂
-O-(CH₂)₂-1-Pyrrolidinyl.

8. Verbindungen der allgemeinen Formel I, worin die Seitenkette -X-E-Y aus der Gruppe der folgenden Seitenketten ausgewählt ist
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅-1-Pyrrolidinyl
-(CH₂)₅N(CH₃)(CH₂)₃OPhenyl
-(CH₂)₅N(CH₃)(CH₂)₃OBenzyl
-(CH₂)₅N(CH₃)(CH₂)₃O(CH₂)₃C₂F₅
-(CH₂)₉S(CH₂)₃C₂F₅
-(CH₂)₉SO(CH₂)₃C₂F₅
-(CH₂)₉SO₂(CH₂)₃C₂F₅

9. Verbindungen der allgemeinen Formel I nach Anspruch 1, worin die Seitenkette -X-E-Y ausgewählt ist aus der allgemeinen Teilformel 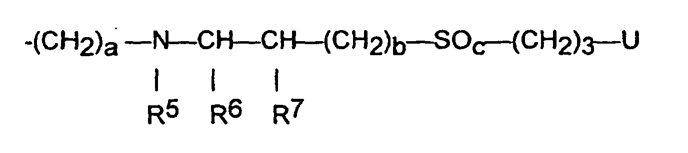 wobei
a 4, 5 oder 6,
b 0, 1 oder 2,
c 0, 1 oder 2,
R⁵ ein Wasserstoffatom oder eine C₁₋₅ Alkylgruppe,
R⁶ und R⁷ je ein Wasserstoffatom, oder
R⁵ und R⁶ gemeinsam eine Alkylengruppe ―(CH₂)_{d}― mit d = 2, 3, 4 oder 5 und R⁷ ein Wasserstoffatom oder
R⁵ und R⁷ gemeinsam eine Alkylengruppe ―(CH₂)ₑ― mit e = 2, 3 oder 4 und R⁶ ein Wasserstoffatom, und
U ein unsubstituierter oder ein- bis fünffach fluorierter Ethylrest ist, oder der terminale Substituent -(CH₂)₃-U in der Seitenkette durch einen gegebenenfalls substituierten Aryl- oder Heteroarylrest, der direkt oder über eine Mono-, Di- oder Trimethylengruppe an das Schwefelatom gebunden ist, ersetzt ist.

10. Verbindungen der allgemeinen Formel I nach Anspruch 9, worin -X-E-Y die Seitenkette -(CH₂)₅N(CH₃)(CH₂)₃S(CH₂)₃C₂F₅ ist.

11. Verbindungen der allgemeinen Formel I nach Anspruch 9, worin -X-E-Y die Seitenkette -(CH₂)₅N(R⁵)(CHR⁶)CH₂S(CH₂)₃C₂F₅ mit R⁵+R⁶ = -(CH₂)₃ist.

12. Verbindungen der allgemeinen Formel 1, nämlich
(1S,3S,4R,6S,9S}-9-Hydroxy-4-(4-hydroxyphenyl)-1-methyl-3-[4-(10,10,11,11,11-pentafluor-6-thia-undecyloxy)phenyl]-bicyclo[4.3.0]nonan
(1S,3S,4R,6S,9S)-9-Acetyloxy-4-(4-hydroxyphenyl)-1-methyl-3-[4-(10,10,11,11,11-pentafluor-6-thia-undecyloxy)phenyl]-bicyclo[4.3.0]nonan
(1S,3S,4R,6S,9S)-9-Hydroxy-4-(4-hydroxyphenyl)-1-methyl-3-[4-(5-(4,4,5,5,5-pentafluor-pentylsulfinyl)pentyloxy)phenyl]-bicyclo[4.3.0]nonan
(1S,3S,4R,6S,9S)-9-Hydroxy-4-(4-hydroxyphenyl)-1-methyl-3-[4-(5-(4,4,5,5,5-pentafluor-pentylsulfonyl)pentyloxy)phenyl]-bicyclo[4.3.0]nonan
(1S,3S,4R,6S,9S)-9-Acetyloxy-4-(4-hydroxyphenyl)-1-methyl-3-[4-(5-(4,4,5,5,5-pentafluor-pentylsulfinyl)pentyloxy)phenyl]-bicyclo[4.3.0]nonan
(1S,6S,9S)-9-Hydroxy-4-(4-hydroxyphenyl)-1-methyl-3-[4-(10,10,11,11,11-pentafluor-6-thia-undecyloxy)phenyl]-bicyclo[4.3.0]non-3-en
(1S,6S,9S)-9-Acetytoxy-4-(4-hydroxyphenyl)-1-methyl-3-[4-(10,10,11,11,11-pentafluor-6-thia-undecyloxy)phenyl]-bicyclo[4.3.0]non-3-en
(1S,6S,9S)-9-Acetyloxy-4-(4-hydroxyphenyl)-1-methyl-3-[4-(5-(4,4,5,5,5-pentafluor-pentylsulfonyl)pentyloxy)phenyl]-bicyclo[4.3.0]non-3-en
(1S,6S,9S)-9-Acetytoxy-4-(4-hydoxyphenyl)-1-methyl-3-[4-(5-(4,4,5,5,5-pentafluor-pentytsulfinyl)pentyloxy)phenyl]-bicyclo[4.3.0]non-3-en
(1S,6S,9S)-9-Hydroxy-4-(4-hydroxyphenyl)-1-methyl-3-[4-(5-(4,4,5,5,5-pentafluor-pentylsulfonyl)pentyloxy)phenyl]-bicyclo[4.3.0]non-3-en
(1S,3S,4R,6S,9S)-3,4-Epoxy-9-hydroxy-4-(4-hydroxyphenyl)-1-methyl-3-[4-(5-(4,4,5,5,5-pentafluor-pentylsulfonyl)pentyloxy)phenyl]-bicyclo[4.3.0]nonan
(1S,6S,9S)-9-Hydroxy-4-(4-hydroxyphenyl)-1-methyl-3-[4-(5-(4,4,5,5,5-pentafluor-pentylsulfinyl)pentyloxy)phenyl]-bicyclo[4.3.0]non-3-en
(1S,3S,4R,6S,9S)-9-Acetoxy-3,4-epoxy-4-(4-hydroxyphenyl)-1-methyl-3-[4-(5-(4,4,5,5,5-pentafluor-pentylsulfonyl)pentyloxy)phenyl]-bicyclo[4.3.0]nonan
(1S,6S,9S)-9-Hydroxy-4-(4-hydroxyphenyl)-1-methyl-3-[4-(2-chlorethyloxy)phenyl]-bicyclo[4.3.0]non-3-en
(1S,6S,9S)-9-Hydroxy-4-(4-hydroxyphenyl)-1-methyl-3-[4-(2-(dimethylamino)ethyloxy)phenyl]-bicyclo[4.3.0]non-3-en
(1S,3S,4R,6S,9S)-9-Hydroxy-4-(4-hydroxyphenyl)-1-methyl-3-[4-(2-(dimethylamino)ethyloxy)phenyl]-bicyclo[4.3.0]nonan
(1S,3S,4R,6S,9S)-9-Acetyloxy-4-(4-hydroxyphenyl)-1-methyl-3-[4-(10,10,11,11,11-pentafluorpentytsulfonyl)pentyloxy)phenyl]-bicyclo[4.3.0]nonan
(1S,3S,4R,6S,9S)-9-Hydroxy-4-(4-hydroxyphenyl)-1-methyl-3-[4-(2-chlorethyloxy)phenyl]-bicyclo[4.3.0]nonan
(1S,3S,4R,6S,9S)-3-[4-(5-Chlorpentyloxy)phenyl]-3,4-epoxy-9-hydroxy-4-(4-hydroxyphenyl)-1-methyl-bicyclo[4.3.0]nonan
(1S,3S,4R,6S,9S)-3-[4-{10,10,11,11,11-pentafluor-6-thia-undecyloxy)phenyl]-3,4-epoxy-9-hydroxy-4-(4-hydroxyphenyl)-1-methyl-bicyclo[4.3.0]nonan.
(1 S,3S,6S,9S)-3-[4-(10,10,11,11,11-pentafluor-6-thia-undecyloxy)phenyl]-3,9-dihydroxy-4-(4-hydroxyphenyl)-1-methyl-bicyclo[4.3.0]non-4-en
(1S,3S,4R,6S,9S)-3-[4-(5-(4,4,5,5,5-pentafluor-pentylsulfinyl)pentyloxy)phenyl-3,4-epoxy-9-hydroxy-4-(4-hydroxyphenyl)-1-methyl-bicyclo[4.3.0]nonan.

13. Verbindungen der allgemeinen Formel nach Anspruch 1, worin R¹ und / oder R² für PG stehen und aus der Gruppe der Substituenten Methoxymethyl-, Methoxyethyl, Ethoxyethyl-, Tetrahydropyranyl-, Tetrahydrofuranyl-. Trimethylsilyl-, Triethylsityl-, tert.-Butyldimethylsilyl-, tert.-Butyldiphenylsilyl-, Tribenzylsilyl-, Triisopropylsilyl-. Methyl-, tert.-Butyl-, Benzyl, para-Nitrobenzyl-, para-Methoxybenzyl-, Formyl, Acetyl-, Propionyl-, Pivaloyl, Butyryl- oder Benzoylrest ausgewählt sind.

14. Diastereomeren und/oder Enantiomere sowie deren Gemische von Verbindungen der allgemeinen Formel I nach Anspruch 1.

15. Verfahren zur Herstellung der Verbindungen der allgemeinen Formel I' 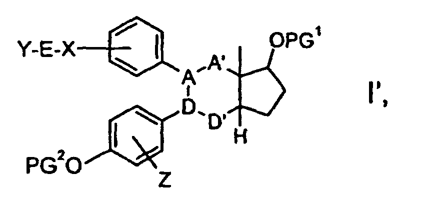 worin
PG¹, PG² gleich oder verschieden sind und für PG, wobei PG ein Methoxymethyl-, Methoxyethyl-, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsityl-, Triisopropylsilyl-. Methyl-, tert.-Butyl-, Benzyl-, para-Nitrobenzyl-, para-Methoxybenzyl, Formyl-, Acetyl-, Propionyl-, Butyryl-, Pivaloyl, Benzoyl-Rest ist,
PG für Wasserstoff oder eine Schutzgruppe,
A'-A-D-D' für eine -CH₂-C(OH)-C=CH-, -CH=C-C(OH)-CH₂-, -CH=C-C=CH-, -CH₂-C=C-CH₂-, -CH₂-CH-CH-CH₂-, -CH₂-C(OH)-CH-CH₂-, -CH₂-CH-C(OH)-CH₂-, -CH₂-C(OH)-C(OH)-CH₂- oder 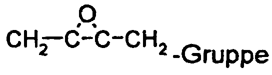 (Hydroxy= α oder β; Epoxy = α oder β),
X für eine Bindung, ein Sauerstoffatom, ein Schwefelatom, SO oder SO₂,
E für eine gerad- oder verzweigtkettige Alkylen-, Alkenylen- oder Alkinylengruppe mit bis zu 15 Kohlenstoffatomen,
Y für Halogen (F, CI, Br, I), für einen Substituenten R⁴, einen gegebenenfalls substituierten Aryl- oder Heteroarylrest, eine NR^{4a}R^{4b}-, SO₂NR^{4a}R^{4b}-, NR^{4a}(CH₂)ₚ-Q-G-, NR⁵(CHR⁶-CHR⁷)-(CH₂)ₜ-Q-G-, SO₂NR^{4a}(CH₂)ₚ-Q-G-, eine O-G-, S-G-. SO-G-, SO₂-G-Gruppe,
R⁴ für ein Wasserstoffatom, gegebenenfalls substituiertes C₁-C₂₀-Alkyl, teilweise oder vollständig fluoriertes C₁-C₂₀-Alkyl, gegebenenfalls substituiertes C₁-C₂₀-Alkanoyl, gegebenenfalls substituiertes Aryl, gegebenenfalls substituiertes Heteroaryl, gegebenenfalls substituiertes C₇-C₂₀-Aralkyl, gegebenenfalls substituiertes C₇-C₁₅-Aroyl,
Q für ein Sauerstoffatom, ein Schwefelatom, SO oder SO₂
G für -(CH₂)ₙ-R³,
n für 0 bis 10,
p für 1 bis 10,
t für 0,1 oder 2
R³ für Wasserstoff, eine gerad- oder verzweigtkettige Alkyl-, Alkenyl- oder Alkinylgruppe mit bis zu 10 Kohlenstoffatomen, eine gerad- oder verzweigtkettige, teilweise oder vollständig fluorierte Alkyl- oder Alkenylgruppe mit bis zu 10 Kohlenstoffatomen, eine gegebenenfalls substituierte C₄-C₈-Cycloalkyl-, eine gegebenenfalls substituierte Aryl-, eine gegebenenfalls substituierte C₇-C₂₀-Aralkylgruppe oder, falls n>0 ist, auch für eine Hydroxygruppe oder ein Halogenatom,
R^{4a}, R^{4b} gleich oder verschieden sind in der Bedeutung von R⁴ oder gemeinsam für eine C₃-C₁₅-Alkylengruppe, die geradkettig oder
R⁵ ein verzweigt sein kann, Wasserstoffatom oder eine C₁₋₅-Alkylgruppe,
R⁶ und R⁷ je ein Wasserstoffatom, oder
R⁵ und R⁶ gemeinsam eine Alkylengruppe ―(CH₂)_{d}― mit d = 2, 3, 4 oder 5 und R⁷ ein Wasserstoffatom oder
R⁵ und R⁷ gemeinsam eine Alkylengruppe ―(CH₂)ₑ― mit e = 2, 3 oder 4 und R⁶ ein Wasserstoffatom,
Z für Wasserstoff, Halogen, OH, N₃, NH₂, CO₂H, CO₂-(C₁-C₂₀)-Alkyl, C₁-C₂₀-Alkoxy, -NO₂, -CN, oder C₁-C₂₀-Acyloxy,
stehen,
**dadurch gekennzeichnet, daß** eine Verbindung der allgemeinen Formel II 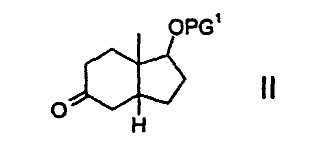 worin PG¹ die in der allgemeinen Formel I angegebene Bedeutung hat, als Ausgangsmaterial verwendet wird.

16. Arzneimittel, enthaltend mindestens eine Verbindung der allgemeinen Formel I gemäß Anspruch 1 sowie einen pharmazeutisch vertägiichen Träger.

17. Arzneimittel nach Anspruch 16, **dadurch gekennzeichnet, daß** die darin enthaltene(n) Verbindung(en) der allgemeinen Formel I als α-, β- oder γ-Cyclodextrinderivat(e) formuliert ist (sind).

18. Arzneimittel nach Anspruch 16, **dadurch gekennzeichnet, daß** die Verbindung(en) der allgemeinen Formel I nach Anspruch 1 mit Liposomen verkapselt ist (sind).

19. Verwendung der Verbindungen der allgemeinen Formel I gemäß Anspruch 1 zur Herstellung von Arzneimitteln.

## Claims

1. 3,4-Diphenylbicyclo[4.3.0]nonyl derivatives of the general formula I 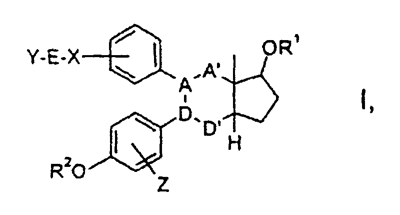 in which
R¹ is optionally substituted C₁-C₂₀-alkanoyl, optionally substituted C₁-C₂₀-alkyl, optionally substituted C₇-C₂₀-aralkyl, optionally substituted C₇-C₁₅-aroyl, a group PG¹ or a hydrogen atom,
R² is optionally substituted C₁-C₂₀-alkanoyl, optionally substituted C₁-C₂₀-alkyl, optionally substituted C₇-C₂₀-aralkyl, optionally substituted C₇-C₁₅-aroyl, a group PG² or a hydrogen atom,
PG¹, PG² are identical or different and are a protective group PG, where PG is a methoxymethyl, methoxyethyl, ethoxyethyl, tetrahydropyranyl, tetrahydrofuranyl, trimethylsilyl, triethylsilyl, tert-butyldimethylsilyl, tert-butyldiphenylsilyl, tribenzylsilyl, triisopropylsilyl, methyl, tert-butyl, benzyl, para-nitrobenzyl, para-methoxybenzyl, formyl, acetyl, propionyl, isopropionyl, butyryl, pivaloyl or benzoyl radical,
A' -A-D-D' is a -CH₂-C (OH)-C=CH-, -CH=C-C(OH)-CH₂-, -CH=C-C=CH-, -CH₂-C=C-CH₂-, -CH₂-CH-CH-CH₂-, -CH₂-C(OH)-CH-CH₂-, -CH₂-CH-C(OH)-CH₂-, -CH₂-C(OH)-C(OH)-CH₂- or CH₂-C-O-C-CH₂ group (hydroxy = α or β); epoxy = α or β),
X is a bond, an oxygen atom, a sulphur atom, SO
E or SO₂, is a straight- or branched-chain alkylene, alkenylene or alkynylene group having up to 15 carbon atoms,
Y is halogen (F, Cl, Br, I), is a substituent R⁴, an optionally substituted aryl or heteroaryl radical, an NR^{4a}R^{4b}, SO₂NR^{4a}R^{4b}, NR^{4a}(CH₂)ₚ-Q-G, NR⁵(CHR⁶-CHR⁷)-(CH₂)ₜ-Q-G, SO₂NR^{4a}(CH₂)ₚ-Q-G, an O-G, S-G, SO-G, SO₂-G group,
R⁴ is a hydrogen atom, optionally substituted C₁-C₂₀-alkyl, partially or completely fluorinated C₁-C₂₀-alkyl, optionally substituted C₁-C₂₀-alkanoyl, optionally substituted aryl, optionally substituted heteroaryl, optionally substituted C₇-C₂₀-aralkyl, optionally substituted C₁-C₁₅-aroyl,
Q is an oxygen atom, a sulphur atom, SO or SO₂
G is -(CH₂)ₙ-R³,
n is 0 to 10,
p is 1 to 10,
t is 0, 1 or 2
R³ is hydrogen, a straight- or branched-chain alkyl, alkenyl or alkynyl group having up to 10 carbon atoms, a straight- or branched-chain, partially or completely fluorinated alkyl or alkenyl group having up to 10 carbon atoms, an optionally substituted C₄-C₈-cycloalkyl, an optionally substituted aryl, an optionally substituted C₇-C₂₀-aralkyl group or, if n is >0, is also a hydroxyl group or a halogen atom,
R^{4a}, R^{4b} are identical or different in the meaning of R⁴ or together are a C₃-C₁₅-alkylene group, which may be straight-chain or branched,
R⁵ is a hydrogen atom or a C₁₋₅-alkyl group,
R⁶ and R⁷ are each a hydrogen atom, or
R⁵ and R⁶ together are an alkylene group -(CH₂)_{d}- with d = 2, 3, 4 or 5 and R⁷ is a hydrogen atom or
R⁵ and R⁷ together are an alkylene group -(CH₂)ₑ- with e = 2, 3 or 4 and R⁶ is a hydrogen atom,
Z is hydrogen, halogen, OH, N₃, NH₂, CO₂H, CO₂-(C₁-C₂₀)-alkyl, C₁₋C₂₀-alkoxy, -NO₂, -CN or C₁-C₂₀-acyloxy.

2. Compounds of the general formula I according to Claim 1, in which R¹ and/or R² are a hydrogen atom.

3. Compounds of the general formula I according to Claim 1, in which A'-A-D-D' is a -CH₂-CH-CH-CH₂- group.

4. Compounds of the general formula I according to Claim 1, in which A'-A-D-D' is a -CH₂-C=C-CH₂- group.

5. Compounds of the general formula I according to Claim 1, in which A'-A-D-D' is a 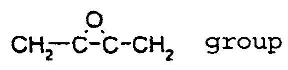 (epoxy = α).

6. Compounds of the general formula I according to Claim 1, in which A'-A-D-D' is a -CH₂-C(OH)-C=CH- group.

7. Compounds of the general formula I, in which the side chain -X-E-Y is selected from the group of the following side chains
-O-(CH₂)₅S(CH₂)₃C₂F₅
-O-(CH₂)₅SO(CH₂)₃C₂F₅
-O-(CH₂)₅SO₂(CH₂)₃C₂F₅
-O-(CH₂)₄S(CH₂)₃C₂F₅
-O-(CH₂)₄SO(CH₂)₃C₂F₅
-O-(CH₂)₄SO₂(CH₂)₃C₂F₅
-O-(CH₂)₅-Cl
-O-(CH₂)₄-Cl
-O-(CH₂)₃-Cl
-O-(CH₂)₂-Cl
-O-(CH₂)₂-N(CH₃)₂
-O-(CH₂)₂-1-pyrrolidinyl.

8. Compounds of the general formula I, in which the side chain -X-E-Y is selected from the group of the following side chains
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅-1-pyrrolidinyl
-(CH₂)₅N(CH₃)(CH₂)₃Ophenyl
-(CH₂)₅N(CH₃)(CH₂)₃Obenzyl
-(CH₂)₅N(CH₃)(CH₂)₃O(CH₂)₃C₂F₅
-(CH₂)₉S(CH₂)₃C₂F₅
-(CH₂)₉SO(CH₂)₃C₂F₅
-(CH₂)₉SO₂(CH₂)₃C₂F₅

9. Compounds of the general formula I according to Claim 1, in which the side chain -X-E-Y is selected from the general partial formula 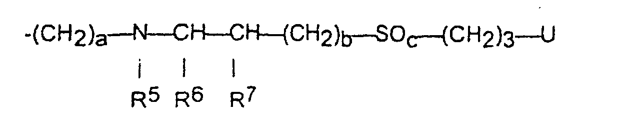 where
a is 4, 5 or 6,
b is 0, 1 or 2,
c is 0, 1 or 2,
R⁵ is a hydrogen atom or a C₁₋₅-alkyl group,
R⁶ and R⁷ are each a hydrogen atom, or
R⁵ and R⁶ together are an alkylene group -(CH₂)_{d}- with d = 2, 3, 4 or 5 and R⁷ a hydrogen atom or R⁵ and R⁷ together are an alkylene group -(CH₂)ₑ-with e = 2, 3 or 4 and R⁶ a hydrogen atom, and
U is an unsubstituted or mono- to pentafluorinated ethyl radical, or the terminal substituent -(CH₂)₃-U in the side chain is replaced by an optionally substituted aryl or heteroaryl radical which is linked directly or via a mono- or di- or trimethylene group to the sulphur atom.

10. Compounds of the general formula I according to Claim 9, in which -X-E-Y is the side chain -(CH₂)₅N(CH₃)(CH₂)₃S(CH₂)₃C₂F₅.

11. Compounds of the general formula I according to Claim 9, in which -X-E-Y is the side chain -(CH₂)₅N(R⁵)(CHR⁶)CH₂S(CH₂)₃C₂F₅ with R⁵+R⁶ = -(CH₂)₃-.

12. Compounds of the general formula I, specifically
(1S,3S,4R,6S,9S)-9-hydroxy-4-(4-hydroxyphenyl)-1-methyl-3-[4-(10,10,11,11,11-pentafluoro-6-thiaundecyloxy)phenyl]bicyclo[4.3.0]nonane
(1S,3S,4R,6S,9S)-9-acetyloxy-4-(4-hydroxyphenyl)-1-methyl-3-[4-(10,10,11,11,11-pentafluoro-6-thiaundecyloxy)phenyl]bicyclo[4.3.0]nonane
(1S,3S,4R,6S,9S)-9-hydroxy-4-(4-hydroxyphenyl)-1-methyl-3-[4-(5-(4,4,5,5,5-pentafluoropentylsulphinyl)-pentyloxy)phenyl]bicyclo[4.3.0]nonane
(1S,3S,4R,6S,9S)-9-hydroxy-4-(4-hydroxyphenyl)-I-methyl-3-[4-(5-(4,4,5,5,5-pentafluoropentylsulphonyl)-pentyloxy)phenyl]bicyclo[4.3.0]nonane
(1S,3S,4R,6S,9S)-9-acetyloxy-4-(4-hydroxyphenyl)-1-methyl-3-[4-(5-(4,4,5,5,5-pentafluoropentylsulphinyl)-pentyloxy)phenyl]bicyclo[4.3.0]nonane
(1S,6S,9S)-9-hydroxy-4-(4-hydroxyphenyl)-1-methyl-3-[4-(10,10,11,11,11-pentafluoro-6-thiaundecyloxy)phenyl]-bicyclo[4.3.0]non-3-ene
(1S,6S,9S)-9-acetyloxy-4-(4-hydroxyphenyl)-1-methyl-3-[4-(10,10,12,11,11-pentafluoro-6-thiaundecyloxy)-phenyl]bicyclo[4.3.0]non-3-ene
(1S,6S,9S)-9-acetyloxy-4-(4-hydroxyphenyl)-1-methyl-3-[4-(5-(4,4,5,5,5-pentafluoropentylsulphonyl)pentyloxy)-phenyl]bicyclo[4.3.0]non-3-ene
(1S,6S,9S)-9-acetyloxy-4-(4-hydroxyphenyl)-1-methyl-3-[4-(5-(4,4,5,5,5-pentafluoropentylsulphinyl)pentyloxy)-phenyl]bicyclo[4.3.0]non-3-ene
(1S,6S,9S)-9-hydroxy-4-(4-hydroxyphenyl)-1-methyl-3-[4-(5-(4,4,5,5,5-pentafluoropentylsulphonyl)pentyloxy)-phenyl]bicyclo[4.3.0]non-3-ene
(1S,3S,4R,6S,9S)-3,4-epoxy-9-hydroxy-4-(4-hydroxyphenyl)-1-methyl-3-[4-(5-(4,4,5,5,5-pentafluoropentylsulphonyl)pentyloxy)phenyl]bicyclo[4.3.0]nonane
(1S,6S,9S)-9-hydroxy-4-(4-hydroxyphenyl)-1-methyl-3-[4-(5-(4,4,5,5,5-pentafluoropentylsulphinyl)pentyloxy)-phenyl]bicyclo[4.3.0]non-3-ene
(1S,3S,4R,6S,9S)-9-acetoxy-3,4-epoxy-4-(4-hydroxyphenyl)-1-methyl-3-[4-(5-(4,4,5,5,5-pentafluoropentylsulphonyl)pentyloxy)phenyl]bicyclo[4.3.0]nonane
(1S,6S,9S)-9-hydroxy-4-(4-hydroxyphenyl}-1-methyl-3-[4-(2-chloroethyloxy)phenyl]bicyclo[4.3.0]non-3-ene
(1S,6S,9S)-9-hydroxy-4-(4-hydroxyphenyl)-1-methyl-3-[4-(2-(dimethylamino)ethyloxy)phenyl]bicyclo[4.3.0]non-3-ene
(1S,3S,4R,6S,9S)-9-hydroxy-4-(4-hydroxyphenyl)-1-methyl-3-[4-(2-(dimethylamino)ethyloxy)phenyl]-bicyclo[4.3.0]nonane
(1S,3S,4R,6S,9S)-9-acetyloxy-4-(4-hydroxyphenyl)-1-methyl-3-[4-(10,10,11,11,11-pentafluoropentylsulphonyl)pentyloxy)phenyl]bicyclo[4.3.0]nonane
(1S,3S,4R,6S,9S)-9-hydroxy-4-(4-hydroxyphenyl)-1-methyl-3-[4-(2-chloroethyloxy)phenyl]bicyclo[4.3.0]-nonane
(1S,3S,4R,6S,9S)-3-[4-(5-chloropentyloxy)phenyl]3,4-epoxy-9-hydroxy-4-(4-hydroxyphenyl)-1-methylbicyclo-[4.3.0]nonane
(1S,3S,4R,6S,9S)-3-[4-(10,10,11,11,11-pentafluoro-6-thiaundecyloxy)phenyl]3,4-epoxy-9-hydroxy-4-(4-hydroxyphenyl)-1-methylbicyclo[4.3.0]nonane
(1S,3S,6S,9S)-3-[4-(10,10,11,11,11-pentafluoro-6-thiaundecyloxy)phenyl]-3,9-dihydroxy-4-(4-hydroxyphenyl)-1-methylbicyclo[4.3.0]non-4-ene
(1S,3S,4R,6S,9S)-3-[4-(5-(4,4,5,5,5-pentafluoropentylsulphinyl)pentyloxy)phenyl-3,4-epoxy-9-hydroxy-4-(4-hydroxyphenyl)-1-methylbicyclo[4.3.0]nonane.

13. Compounds of the general formula I according to Claim 1, in which R¹ and/or R² are PG and are selected from the group of substituents methoxymethyl, methoxyethyl, ethoxyethyl, tetrahydropyranyl, tetrahydrofuranyl, trimethylsilyl, triethylsilyl, tert-butyldimethylsilyl, tert-butyldiphenylsilyl, tribenzylsilyl, triisopropylsilyl, methyl, tert-butyl, benzyl, para-nitrobenzyl, para-methoxybenzyl, formyl, acetyl, propionyl, isopropionyl, pivaloyl, butyryl or benzoyl radicals.

14. Diastereomers and/or enantiomers, and mixtures thereof, of compounds of the general formula I according to Claim 1.

15. Process for the preparation of the compounds of the general formula I' 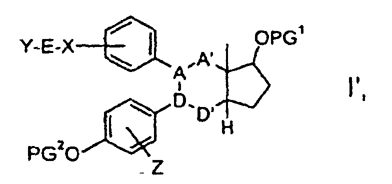 in which
PG¹, PG² are identical or different and are PG, where PG is a methoxymethyl, methoxyethyl, ethoxyethyl, tetrahydropyranyl, tetrahydrofuranyl, trimethylsilyl, triethylsilyl, tert-butyldimethylsilyl, tert-butyldiphenylsilyl, tribenzylsilyl, triisopropylsilyl, methyl, tert-butyl, benzyl, para-nitrobenzyl, para-methoxybenzyl, formyl, acetyl, propionyl, isopropionyl, butyryl, pivaloyl or benzoyl radical,
PG is hydrogen or a protective group,
A'-A-D-D' is a -CH₂-C(OH)-C=CH-, -CH=C-C(OH)-CH₂-, -CH=C-C=CH-, -CH₂-C=C-CH₂-, -CH₂-CH-CH-CH₂-, -CH₂-C(OH)-CH-CH₂-, -CH₂-CH-C(OH)-CH₂-, -CH₂-C(OH)-C(OH)-CH₂- or 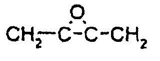 (hydroxy = α or β); epoxy = α or β),
X is a bond, an oxygen atom, a sulphur atom, SO or SO₂,
E is a straight- or branched-chain alkylene, alkenylene or alkynylene group having up to 15 carbon atoms,
Y is halogen (F, Cl, Br, I), is a substituent R⁴, an optionally substituted aryl or heteroaryl radical, an NR^{4a}R^{4b}, SO₂NR^{4a}R^{4b}, NR^{4a} (CH₂)ₚ-Q-G, NR⁵(CHR⁶-CHR⁷)-(CH₂)ₜ-Q-G, SO₂NR^{4a}(CH₂)ₚ-Q-G, an O-G, S-G, SO-G, SO₂-G group,
R⁴ is a hydrogen atom, optionally substituted C₁-C₂₀-alkyl, partially or completely fluorinated C₁-C₂₀-alkyl, optionally substituted C₁-C₂₀-alkanoyl, optionally substituted aryl, optionally substituted heteroaryl, optionally substituted C₇-C₂₀-aralkyl, optionally substituted C₇-C₁₅-aroyl,
Q is an oxygen atom, a sulphur atom, SO or SO₂
G is -(CH₂)ₙ-R³,
n is 0 to 10,
p is 1 to 10,
t is 0, 1 or 2
R³ is hydrogen, a straight- or branched-chain alkyl, alkenyl or alkynyl group having up to 10 carbon atoms, a straight- or branched-chain, partially or completely fluorinated alkyl or alkenyl group having up to 10 carbon atoms, an optionally substituted C₄-C₈-cycloalkyl, an optionally substituted aryl, an optionally substituted C₇-C₂₀-aralkyl group or, if n is >0, is also a hydroxyl group or a halogen atom,
R^{4a}, R^{4b} are identical or different in the meaning of R⁴ or together are a C₃-C₁₅-alkylene group, which may be straight-chain or branched,
R⁵ is a hydrogen atom or a C₁₋₅-alkyl group,
R⁶ and R⁷ are each a hydrogen atom, or
R⁵ and R⁶ together are an alkylene group -(CH₂)_{d}- with d = 2, 3, 4 or 5 and R⁷ is a hydrogen atom or
R⁵ and R⁷ together are an alkylene group -(CH₂)ₑ- with e = 2, 3 or 4 and R⁶ is a hydrogen atom,
Z is hydrogen, halogen, OH, N₃, NH₂, CO₂H, CO₂-(C₁-C₂₀)-alkyl, C₁-C₂₀-alkoxy, -NO₂, -CN or C₁-C₂₀-acyloxy,
**characterized in that** a compound of the general formula II 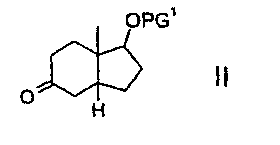 in which PG¹ has the meaning stated in the general formula I is used as starting material.

16. Medicament comprising at least one compound of the general formula I according to Claim 1, and a pharmaceutically acceptable carrier.

17. Medicament according to Claim 16, **characterized in that** the compound(s) of the general formula I contained therein is (are) formulated as α-, β- or γ-cyclodextrin derivative(s).

18. Medicament according to Claim 16, **characterized in that** the compound(s) of the general formula I according to Claim 1 is (are) encapsulated with liposomes.

19. Use of the compounds of the general formula I according to Claim 1 for producing medicaments.

## Revendications

1. Dérivés 3,4-diphényl-bicyclo[4.3.0]nonyle de formule générale I 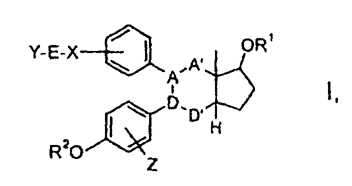 dans laquelle
R¹ représente un alcanoyle en C₁-C₂₀ éventuellement substitué, un alkyle en C₁-C₂₀ éventuellement substitué, un aralkyle en C₇-C₂₀ éventuellement substitué, un aroyle en C₇-C₁₅ éventuellement substitué, un groupe PG¹ ou un atome d'hydrogène,
R² représente un alcanoyle en C₁-C₂₀ éventuellement substitué, un alkyle en C₁-C₂₀ éventuellement substitué, un aralkyle en C₇-C₂₀ éventuellement substitué, un aroyle en C₇-C₁₅ éventuellement substitué, un groupe PG² ou un atome d'hydrogène,
PG¹, PG² sont identiques ou différents et représentent un groupe protecteur PG, où PG représente un radical méthoxyméthyle, méthoxyéthyle, éthoxyéthyle, tétrahydropyranyle, tétrahydrofuranyle, triméthylsilyle, triéthylsilyle, tert-butyldiméthylsilyle, tert-butyldiphénylsilyle, tribenzylsilyle, triisopropylsilyle, méthyle, tert-butyle, benzyle, para-nitrobenzyle, para-méthoxybenzyle, formyle, acétyle, propionyle, isopropionyle, butyryle, pivalyle, benzoyle,
A'-A-D-D' représente un groupe -CH₂-C(OH)-C=CH-, -CH=C-C(OH)-CH₂-, -CH=C-C=CH-, -CH₂-C=C-CH₂-, -CH₂-CH-CH-CH₂-, -CH₂-C(OH)-CH-CH₂-, -CH₂-CH-C(OH)-CH₂-,
-CH₂-C(OH)-C(OH)-CH₂- ou 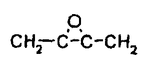 (hydroxy = α ou β ; époxy = α ou β),
X représente une liaison, un atome d'oxygène, un atome de soufre, SO ou SO₂,
E représente un groupe alkylène, alcénylène ou alcynylène linéaire ou ramifié ayant jusqu'à 15 atomes de carbone,
Y représente un halogène (F, Cl, Br, I), un substituant R⁴, un radical aryle ou hétéroaryle éventuellement substitué, un groupe NR^{4a}R^{4b}- , SO₂NR^{4a}R^{4b}- , NR^{4a}(CH₂)ₚ-Q-G-, NR⁵(CHR⁶-CHR⁷)-(CH₂)ₜ-Q-G-, SO₂NR^{4a}(CH₂)ₚ-Q-G-, O-G-, S-G-, SO-G-, SO₂-G-,
R⁴ représente un atome d'hydrogène, un alkyle en C₁-C₂₀ éventuellement substitué, un alkyle en C₁-C₂₀ partiellement ou totalement fluoré, un alcanoyle en C₁-C₂₀ éventuellement substitué, un aryle éventuellement substitué, un hétéroaryle éventuellement substitué, un aralkyle en C₇-C₂₀ éventuellement substitué, un aroyle en C₇-C₁₅ éventuellement substitué,
Q représente un atome d'oxygène, un atome de soufre, SO ou SO₂
G représente -(CH₂)ₙ-R³,
n vaut de 0 à 10,
p vaut de 1 à 10,
t vaut 0, 1 ou 2,
R³ représente un hydrogène, un groupe alkyle, alcényle ou alcynyle linéaire ou ramifié ayant jusqu'à 10 atomes de carbone, un groupe alkyle ou alcényle linéaire ou ramifié, partiellement ou totalement fluoré, ayant jusqu'à 10 atomes de carbone, un groupe cycloalkyle en C₄-C₈ éventuellement substitué, un groupe aryle éventuellement substitué, un groupe aralkyle en C₇-C₂₀ éventuellement substitué ou, dans le cas où n > 0, également un groupe hydroxy ou un atome d'halogène,
R^{4a} R^{4b} sont identiques ou différents pour la signification de R⁴ ou représentent conjointement un groupe alkylène en C₃-C₁₅, qui peut être linéaire ou ramifié,
R⁵ représente un atome d'hydrogène ou un groupe alkyle en C₁-C₅,
R⁶ et R⁷ représentent chacun un atome d'hydrogène, ou
R⁵ et R⁶ représentent conjointement un groupe alkylène -(CH₂)_{d}- dans lequel d = 2, 3, 4 ou 5 et R⁷ représente un atome d'hydrogène, ou
R⁵ et R⁷ représentent conjointement un groupe alkylène -(CH₂)ₑ- dans lequel e = 2, 3 ou 4 et R⁶ représente un atome d'hydrogène,
Z représente un hydrogène, un halogène, OH, N₃, NH₂, CO₂H, un CO₂-(C₁-C₂₀)-alkyle, un alcoxy en C₁-C₂₀, -NO₂, -CN ou un acyloxy en C₁-C₂₀.

2. Composés de formule générale I selon la revendication 1, dans laquelle R¹ et/ou R² représentent un atome d'hydrogène.

3. Composés de formule générale I selon la revendication 1, dans laquelle A'-A-D-D' représente un groupe -CH₂-CH-CH-CH₂-.

4. Composés de formule générale I selon la revendication 1, dans laquelle A'-A-D-D' représente un groupe -CH₂-C=C-CH₂-.

5. Composés de formule générale I selon la revendication 1, dans laquelle A'-A-D-D' représente un groupe 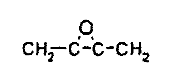 (époxy = α) .

6. Composés de formule générale I selon la revendication 1, dans laquelle A'-A-D-D' représente un groupe -CH₂-C(OH)-C=CH-.

7. Composés de formule générale I, dans laquelle la chaîne latérale -X-E-Y est choisie parmi le groupe des chaînes latérales suivantes
-O-(CH₂)₅S(CH₂)₃C₂F₅
-O-(CH₂)₅SO(CH₂)₃C₂F₅
-O-(CH₂)₅SO₂(CH₂)₃C₂F₅
-O-(CH₂)₄S(CH₂)₃C₂F₅
-O-(CH₂)₄SO(CH₂)₃C₂F₅
-O-(CH₂)₄SO₂(CH₂)₃C₂F₅
-O-(CH₂)₅-Cl
-O-(CH₂)₄-Cl
-O-(CH₂)₃-Cl
-O-(CH₂)₂-Cl
-O-(CH₂)₂*-*N(CH₃)₂
-O-(CH₂)₂-1-pyrrolidinyle.

8. Composés de formule générale I, dans laquelle la chaîne latérale -X-E-Y est choisie parmi le groupe des chaînes latérales suivantes
-(CH₂)₅N(CH₃)(CH₂)₃C₂F₅
-(CH₂)₅N(CH₃)(CH₂)₆C₂F₅
-(CH₂)₅N(CH₃)(CH₂)₇C₂F₅
-(CH₂)₅N(CH₃)(CH₂)₈C₂F₅
-(CH₂)₆N(CH₃)(CH₂)₆C₂F₅
-(CH₂)₆N(CH₃)(CH₂)₇C₂F₅
-(CH₂)₆N(CH₃)(CH₂)₈C₂F₅
-(CH₂)₅N(CH₃)(CH₂)₂C₄F₉
-(CH₂)₅N(CH₃)(CH₂)₃C₆F₁₃
-(CH₂)₅N(CH₃)(CH₂)₃C₈F₁₇
-(CH₂)₅N(CH₃)(CH₂)₆C₄F₉
-(CH₂)₅N(CH₃)(CH₂)₆C₆F₁₃
-(CH₂)₅N(CH₃)(CH₂)₆C₈F₁₇
-(CH₂)₅N(CH₃)H
-(CH₂)₅N(CH₃)(CH₂)₉H
-(CH₂)₅-1-pyrrolidinyle
-(CH₂)₅N(CH₃)(CH₂)₃Ophényle
-(CH₂)₅N(CH₃)(CH₂)₃Obenzyle
-(CH₂)₅N(CH₃)(CH₂)₃O(CH₂)₃C₂F₅
-(CH₂)₉S(CH₂)₃C₂F₅
-(CH₂)₉SO(CH₂)₃C₂F₅
-(CH₂)₉SO₂ (CH₂)₃C₂F₅.

9. Composés de formule générale I selon la revendication 1, dans laquelle la chaîne latérale -X-E-Y est choisie parmi la formule partielle générale 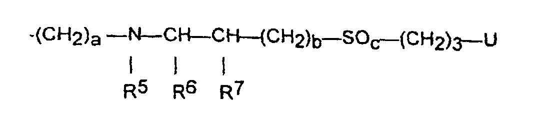 dans laquelle
a vaut 4, 5 ou 6,
b vaut 0, 1 ou 2,
c vaut 0, 1 ou 2,
R⁵ représente un atome d'hydrogène ou un groupe alkyle en C₁₋₅,
R⁶ et R⁷ représentent chacun un atome d'hydrogène, ou
R⁵ et R⁶ représentent conjointement un groupe alkylène -(CH₂)_{d}- dans lequel d = 2, 3, 4 ou 5 et R⁷ représente un atome d'hydrogène, ou
R⁵ et R⁷ représentent conjointement un groupe alkylène -(CH₂)ₑ- dans lequel e = 2, 3 ou 4 et R⁶ représente un atome d'hydrogène, et
U représente un radical éthyle non substitué ou mono- à penta-fluoré, ou le substituant terminal -(CH₂)₃-U dans la chaîne latérale a été remplacé par un radical aryle ou hétéroaryle éventuellement substitué, qui est lié à l'atome de soufre directement ou par l'intermédiaire d'un groupe mono-, di- ou triméthylène.

10. Composés de formule générale I selon la revendication 9, dans laquelle -X-E-Y est la chaîne latérale -(CH₂)₅N(CH₃)(CH₂)₃S(CH₂)₃C₂F₅.

11. Composés de formule générale I selon la revendication 9, dans laquelle -X-E-Y est la chaîne latérale -(CH₂)₅N(R⁵)(CHR⁶)CH₂S(CH₂)₃C₂F₅ dans laquelle R⁵ + R⁶ = -(CH₂)₃-.

12. Composés de formule générale I, à savoir
le (1S,3S,4R,6S,9S)-9-hydroxy-4-(4-hydroxyphényl)-1-méthyl-3-[4-(10,10,11,11,11-pentafluoro-6-thia-undécyloxy)phényl]bicyclo[4.3.0]nonane
le (1S,3S,4R,6S,9S)-9-acétyloxy-4-(4-hydroxyphényl)-1-méthyl-3-[4-(10,10,11,11,11-pentafluoro-6-thia-undécyloxy)phényl]bicyclo[4.3.0]nonane
le (1S,3S,4R,6S,9S)-9-hydroxy-4-(4-hydroxyphényl)-1-méthyl-3-[4-(5-(4,4,5,5,5-pentafluoro-pentylsulfinyl)pentyloxy)phényl]bicyclo[4.3.0]nonane
le (1S,3S,4R,6S,9S)-9-hydroxy-4-(4-hydroxyphényl)-1-méthyl-3-[4-(5-(4,4,5,5,5-pentafluoropentylsulfonyl)pentyloxy)phényl]bicyclo[4.3.0]nonane
le (1S,3S,9R,6S,9S)-9-acétyloxy-4-(4-hydroxyphényl)-1-méthyl-3-[4-(5-(4,4,5,5,5-pentafluoropentylsulfinyl)pentyloxy)phényl]bicyclo[4.3.0]nonane
le (1S,6S,9S)-9-hydroxy-4-(4-hydroxyphényl)-1-méthyl-3-[4-(10,10,11,11,11-pentafluoro-6-thia-undécyloxy)phényl]bicyclo[4.3.0]non-3-ène
le (1S,6S,9S)-9-acétyloxy-4-(4-hydroxyphényl)-1-méthyl-3-[4-(10,10,11,11,11-pentafluoro-6-thia-undécyloxy)phényl]bicyclo[4.3.0]non-3-ène
le (1S,6S,9S)-9-acétyloxy-4-(4-hydroxyphényl)-1-méthyl-3-[4-(5-(4,4,5,5,5-pentafluoropentylsulfonyl)pentyloxy)phényl]bicyclo[4.3.0]non-3-ène
le (1S,6S,9S)-9-acétyloxy-4-(4-hydroxyphényl)-1-méthyl-3-[4-(5-(4,4,5,5,5-pentafluoropentylsulfinyl)pentyloxy)phényl]bicyclo[4.3.0]non-3-ène
le (1S,6S,9S)-9-hydroxy-4-(4-hydroxyphényl)-1-méthyl-3-[4-(5-(4,4,5,5,5-pentafluoropentylsulfonyl)pentyloxy)phényl]bicyclo[4.3.0]non-3-ène
le (1S,3S,4R,6S,9S)-3,4-époxy-9-hydroxy-4-(4-hydroxyphényl)-1-méthyl-3-[4-(5-(4,4,5,5,5-pentafluoropentylsulfonyl)pentyloxy)phényl]bicyclo[4.3.0]nona ne
le (1S,6S,9S)-9-hydroxy-4-(4-hydroxyphényl)-1-méthyl-3-[4-(5-(4,4,5,5,5-pentafluoropentylsulfinyl)pentyloxy)phényl]bicyclo [4.3.0] non3-ène
le (1S,3S,4R,6S,9S)-9-acétoxy-3,4-époxy-4-(4-hydroxyphényl)-1-méthyl-3-[4-(5-(4,4,5,5,5-pentafluoropentylsulfonyl)pentyloxy)phényl]bicyclo[4.3.0]nonane
le (1S,6S,9S)-9-hydroxy-4-(4-hydroxyphényl)-1-méthyl-3-[4-(2-chloroéthyloxy)phényl]bicyclo[4.3.0]non-3-ène
le (1S,6S,9S)-9-hydroxy-4-(4-hydroxyphényl)-1-méthyl-3-[4-(2-(diméthylamino)éthyloxy)phényl]bicyclo[4.3.0]non-3-ène
le (1S,3S,4R,6S,9S)-9-hydroxy-4-(4-hydroxyphényl)-1-méthyl-3-[4-(2-(diméthylamino)éthyloxy)phényl]bicyclo[4.3.0]nonane
le (1S,3S,4R,6S,9S)-9-acétyloxy-4-(4-hydroxyphényl)-1-méthyl-3-[4-(10,10,11,11,11-pentafluoropentylsulfonyl)pentyloxy)phényl]bicyclo [4.3.0]nonane
le (1S,3S,4R,6S,9S)-9-hydroxy-4-(4-hydroxyphényl)-1-méthyl-3-[4-(2-chloroéthyloxy)phényl]bicyclo[4.3.0]nonane
le (1S,3S,4R,6S,9S)-3-[4-(5-chloropentyloxy)phényl]-3,4-époxy-9-hydroxy-4-(4-hydroxyphényl)-1-méthyl-bicyclo[4.3.0]nonane
le (1S,3S,4R,6S,9S)-3-[4-(10,10,11,11,11-pentafluoro-6-thia-undécyloxy)phényl]-3,4-époxy-9-hydroxy-4-(4-hydroxyphényl)-1-méthylbicyclo[4.3.0]nonane
le (1S,3S,6S,9S)-3-[4-(10,10,11,11,11-pentafluoro-6-thia-undécyloxy)phényl]-3,9-dihydroxy-4-(4-hydroxyphényl)-1-méthyl-bicyclo[4.3.0]non-4-ène
le (1S,3S,4R,6S,9S)-3-[4-(5-(4,4,5,5,5-pentafluoro-pentylsulfinyl)pentyloxy)phényl-3,4-époxy-9-hydroxy-4-(4-hydroxyphényl)-1-méthylbicyclo [4.3.0] nonane.

13. Composés de formule générale I selon la revendication 1, dans laquelle R¹ et / ou R² représentent PG et sont choisis parmi le groupe des substituants constitués des radicaux méthoxyméthyle, méthoxyéthyle, éthoxyéthyle, tétrahydropyranyle, tétrahydrofuranyle, triméthylsilyle, triéthylsilyle, tert-butyldiméthylsilyle, tert-butyldiphénylsilyle, tribenzylsilyle, triisopropylsilyle, méthyle, tert-butyle, benzyle, para-nitrobenzyle, para-méthoxybenzyle, formyle, acétyle, propionyle, isopropionyle, pivalyle, butyryle,ou benzoyle.

14. Diastéréoisomères et/ou énantiomères ainsi que leurs mélanges de composés de formule générale I selon la revendication 1.

15. Procédé de préparation des composés de formule générale I' 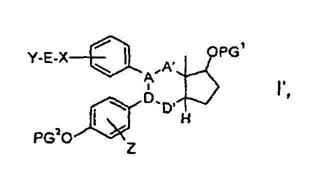 dans laquelle
PG¹, PG² sont identiques ou différents et représentent PG, où PG représente un radical méthoxyméthyle, méthoxyéthyle, éthoxyéthyle, tétrahydropyranyle, tétrahydrofuranyle, triméthylsilyle, triéthylsilyle, tert-butyldiméthylsilyle, tert-butyldiphénylsilyle, tribenzylsilyle, triisopropylsilyle, méthyle, tert-butyle, benzyle, para-nitrobenzyle, para-méthoxybenzyle, formyle, acétyle, propionyle, isopropionyle, butyryle, pivalyle, benzoyle,
PG représente un hydrogène ou un groupe protecteur,
A'-A-D-D' représente un groupe -CH₂-C(OH)-C=CH-, -CH=C-C(OH)-CH₂-, -CH=C-C=CH-, -CH₂-C=C CH₂-, -CH₂-CH-CH-CH₂-, -CH₂-C(OH)-CH-CH₂-, -CH₂-CH-C(OH)-CH₂-,
-CH₂-C(OH)-C(OH)-CH₂- ou 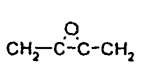 (hydroxy = α ou ; époxy = α ou β),
X représente une liaison, un atome d'oxygène, un atome de soufre, SO ou SO₂,
E représente un groupe alkylène, alcénylène ou alcynylène linéaire ou ramifié ayant jusqu'à 15 atomes de carbone,
Y représente un halogène (F, Cl, Br, I), un substituant R⁴, un radical aryle ou hétéroaryle éventuellement substitué, un groupe NR^{4a}R^{4b}- , SO₂NR^{4a}R^{4b}-, NR^{4a}(CH₂)ₚ-Q-G-, NR⁵(CHR⁶-CHR⁷)-(CH₂)ₜ-Q-G-, SO₂NR^{4a}(CH₂)ₚ-Q-G-, O-G-, S-G-, SO-G-, SO₂-G-,
R⁴ représente un atome d'hydrogène, un alkyle en C₁-C₂₀ éventuellement substitué, un alkyle en C₁-C₂₀ partiellement ou totalement fluoré, un alcanoyle en C₁-C₂₀ éventuellement substitué, un aryle éventuellement substitué, un hétéroaryle éventuellement substitué, un aralkyle en C₇-C₂₀ éventuellement substitué, un aroyle en C₇-C₁₅ éventuellement substitué,
Q représente un atome d'oxygène, un atome de soufre, SO ou SO₂,
G représente -(CH₂)ₙ-R³,
n vaut de 0 à 10,
p vaut de 1 à 10,
t vaut 0, 1 ou 2,
R³ représente un hydrogène, un groupe alkyle, alcényle ou alcynyle linéaire ou ramifié ayant jusqu'à 10 atomes de carbone, un groupe alkyle ou alcényle linéaire ou ramifié, partiellement ou totalement fluoré, ayant jusqu'à 10 atomes de carbone, un groupe cycloalkyle en C₄-C₈ éventuellement substitué, un groupe aryle éventuellement substitué, un groupe aralkyle en C₇-C₂₀ éventuellement substitué ou, dans le cas où n > 0, également un groupe hydroxy ou un atome d'halogène,
R^{4a}, R^{4b} sont identiques ou différents pour la signification de R⁴ ou représentent conjointement un groupe alkylène en C₃-C₁₅, qui peut être linéaire ou ramifié,
R⁵ représente un atome d'hydrogène ou un groupe alkyle en C₁-C₅,
R⁶ et R⁷ représentent chacun un atome d'hydrogène, ou
R⁵ et R⁶ représentent conjointement un groupe alkylène -(CH₂)_{d}- dans lequel d = 2, 3, 4 ou 5 et R⁷ représente un atome d'hydrogène, ou
R⁵ et R⁷ représentent conjointement un groupe alkylène -(CH₂)ₑ- dans lequel e = 2, 3 ou 4 et R⁶ représente un atome d'hydrogène,
Z représente un hydrogène, un halogène, OH, N₃, NH₂, CO₂H, un CO₂-(C₁-C₂₀)-alkyle, un alcoxy en C₁-C₂₀, -NO₂, -CN ou un acyloxy en C₁-C₂₀,
**caractérisé en ce qu'**un composé de formule générale II 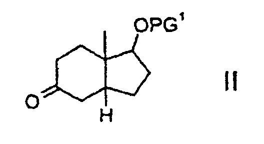 dans laquelle PG¹ présente la signification indiquée dans la formule générale I, est utilisé en tant que substance de départ.

16. Médicament comprenant au moins un composé de formule générale I selon la revendication 1 ainsi qu'un support pharmaceutiquement acceptable.

17. Médicament selon la revendication 16, **caractérisé en ce que** le(s) composé(s) qui y est (sont) contenu(s) de formule générale I est (sont) formulé (s) sous forme de dérivé (s) de α-, β- ou γ-cyclodextrine.

18. Médicament selon la revendication 16, **caractérisé en ce que** le(s) composé(s) de formule générale I selon la revendication 1 est (sont) encapsulé(s) avec des liposomes.

19. Utilisation des composés de formule générale I selon la revendication 1 pour la préparation de médicaments.
